# EUROPEAN PATENT SPECIFICATION

(11) **EP 2 519 300 B1**
(45) Date of publication and mention of the grant of the patent: **12.08.2020**
(21) Application number: 10801347.5
(22) Date of filing: 27.12.2010
(51) Int. Cl.: A61M 25/00, A61M 25/06, A61M 25/10, A61M 29/00, A61B 17/3207, A61L 29/00, A61K 31/00

(54) **SYSTEM FOR TREATING TARGET TISSUE WITHIN THE EUSTACHIAN TUBE**
SYSTEM ZUR BEHANDLUNG VON ZIELGEWEBE IN DER EUSTACHISCHEN RÖHRE
SYSTÈME DE TRAITEMENT D'UN TISSU CIBLE DANS LA TROMPE D'EUSTACHE

(30) Priority: 29.12.2009 US 649078
(43) Date of publication of application: 07.11.2012
(73) Proprietor: Acclarent, Inc., Irvine, CA 92618 (US)
(72) Inventor: CLIFFORD, Anton, G., Mountain View California 94040 (US); MAKOWER, Joshua, Los Altos California 94022 (US); CHANG, John, Y., Los Altos, CA 94022 (US); MORRISS, John, H., San Francisco California 94107 (US); BRIGHT, Earl, A., Los Altos California 94022 (US); GOLDFARB, Eric, Belmont California 94002 (US); VRANY, Julia, D., Sunnyvale California 94086 (US)
(74) Representative: Tunstall, Christopher Stephen
(86) International application number: PCT/US2010/062161
(87) International publication number: WO 2011/082139

(56) References cited:
- WO-A2-2006/078884
- US-A1- 2007 135 789
- US-A1- 2009 163 890
- US-A1- 2010 198 191

## Description

### CROSS-REFERENCES TO RELATED APPLICATIONS

### BACKGROUND OF THE INVENTION

The present invention is related to systems for accessing, diagnosing and treating target tissue regions within the middle ear and the Eustachian tube.

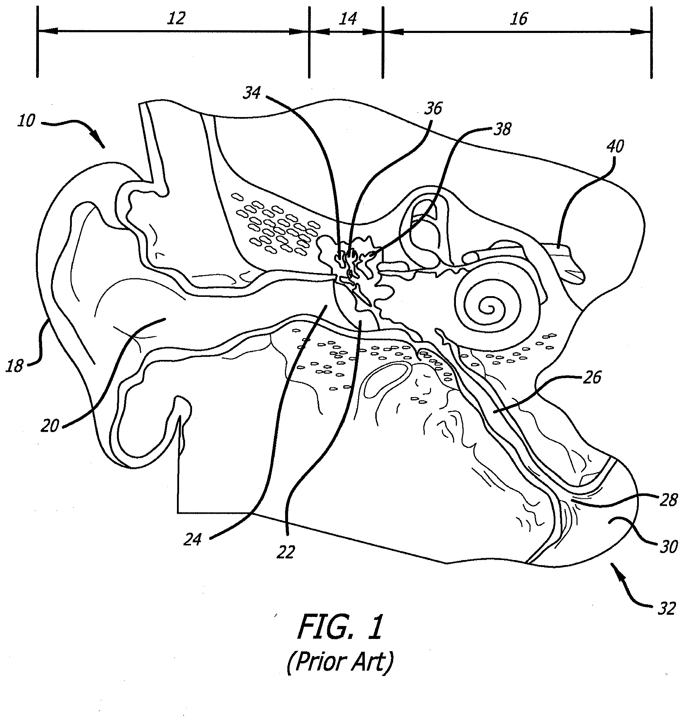

Referring to Figs. 1-2, the ear 10 is divided into three parts: an external ear 12, a middle ear 14 and an inner ear 16. The external ear 12 consists of an auricle 18 and ear canal 20 that gather sound and direct it towards a tympanic membrane 22 (also referred to as the eardrum) located at an inner end 24 of the ear canal 20. The middle ear 14 lies between the external and inner ears 12 and 16 and is connected to the back of the throat by a Eustachian tube 26 which serves as a pressure equalizing valve between the ear 10 and the sinuses. The Eustachian tube 26 terminates in a distal opening 28 in the nasopharynx region 30 of the throat 32. In addition to the eardrum 22, the middle ear 14 also consists of three small ear bones (ossicles): the malleus 34 (hammer), incus 36 (anvil) and stapes 38 (stirrup). These bones 34-38 transmit sound vibrations to the inner ear 16 and thereby act as a transformer, converting sound vibrations in the canal 20 of the external ear 12 into fluid waves in the inner ear 16. These fluid waves stimulate several nerve endings 40 that, in turn, transmit sound energy to the brain where it is interpreted.

The Eustachian tube 26 is a narrow, one-and-a-half inch long channel connecting the middle ear 14 with the nasopharynx 30, the upper throat area just above the palate, in back of the nose. The Eustachian tube 26 functions as a pressure equalizing valve for the middle ear 14 which is normally filled with air. When functioning properly, the Eustachian tube 26 opens for a fraction of a second periodically (about once every three minutes) in response to swallowing or yawning. In so doing, it allows air into the middle ear 14 to replace air that has been absorbed by the middle ear lining (mucous membrane) or to equalize pressure changes occurring on altitude changes. Anything that interferes with this periodic opening and closing of the Eustachian tube 26 may result in hearing impairment or other ear symptoms.

Obstruction or blockage of the Eustachian tube 26 results in a negative middle ear pressure 14, with retraction (sucking in) of the eardrum 22. In adults, this is usually accompanied by some ear discomfort, a fullness or pressure feeling and may result in a mild hearing impairment and head noise (tinnitus). There may be no symptoms in children. If the obstruction is prolonged, fluid may be drawn from the mucous membrane of the middle ear 14, creating a condition referred to as serous otitis media (fluid in the middle ear). This occurs frequently in children in connection with an upper respiratory infection and accounts for the hearing impairment associated with this condition.

A lining membrane (mucous membrane) of the middle ear 14 and Eustachian tube 26 is connected with, and is the same as, the membrane of the nose 42, sinuses 44 and throat 32. Infection of these areas results in mucous membrane swelling which in turn may result in obstruction of the Eustachian tube 26. This is referred to as serous otitis media, i.e. essentially a collection of fluid in the middle ear 14 that can be acute or chronic, usually the result of blockage of the distal opening 28 of the Eustachian tube 26 which allows fluid to accumulate in the middle ear 14. In the presence of bacteria, this fluid may become infected, leading to an acute suppurative otitis media (infected or abscessed middle ear). When infection does not develop, the fluid remains until the Eustachian tube 26 again begins to function normally, at which time the fluid is absorbed or drains down the tube into the throat 32 through the Eustachian tube opening 28.

Chronic serous otitis media may result from longstanding Eustachian tube blockage, or from thickening of the fluid so that it cannot be absorbed or drained down the Eustachian tube 26. This chronic condition is usually associated with hearing impairment. There may be recurrent ear pain, especially when the individual catches a cold. Fortunately, serous otitis media may persist for many years without producing any permanent damage to the middle ear mechanism. The presence of fluid in the middle ear 14, however, makes it very susceptible to recurrent acute infections. These recurrent infections may result in middle ear damage.

When the Eustachian tube 26 contains a build-up of fluid, a number of things will occur. First, the body absorbs the air from the middle ear 14, causing a vacuum to form which tends to pull the lining membrane and ear drum 22 inward, causing pain. Next, the body replaces the vacuum with more fluid which tends to relieve the pain, but the patient can experience a fullness sensation in the ear 10. Treatment of this condition with antihistamines and decongestants can take many weeks to be fully effective. Finally, the fluid can become infected, which is painful and makes the patient feel ill and which may cause the patient not to be able to hear well. If the inner ear 14 is affected, the patient may feel a spinning or turning sensation (vertigo). The infection is typically treated with antibiotics.

However, even if antihistamines, decongestants and antibiotics are used to treat an infection or other cause of fluid build-up in the middle ear 14, these treatments will typically not immediately resolve the pain and discomfort caused by the buildup of fluid in the middle ear 14; i.e. the most immediate relief will be felt by the patient if the fluid can be removed from the Eustachian tube 26.

Antibiotic treatment of middle ear infections typically results in normal middle ear function within three to four weeks. During the healing period, the patient can experience varying degrees of ear pressure, popping, clicking and fluctuation of hearing, occasionally with shooting pain in the ear. Resolution of the infection occasionally leaves the patient with uninfected fluid in the middle ear 14, localized in the Eustachian tube 26.

Fluid build-up caused by these types of infections has been treated surgically in the past. The primary objective of surgical treatment of chronic serous otitis media is to reestablish ventilation of the middle ear, keeping the hearing at a normal level and preventing recurrent infection that might damage the eardrum membrane and middle ear bones.

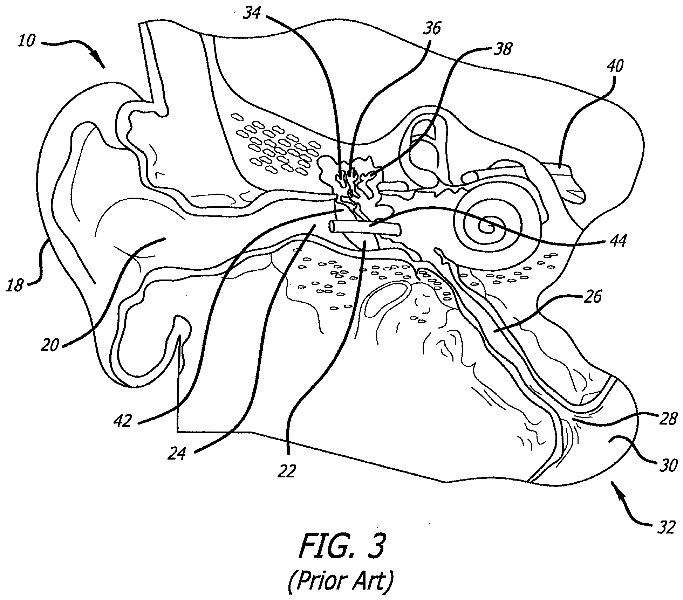

For example, as shown in Fig. 3, a myringotomy can be performed to relieve fluid in the middle ear 14. A myringotomy is an incision 42 in the eardrum 22 performed to remove fluid in the middle ear 14. A hollow plastic tube 44, referred to as a ventilation tube, is inserted and lodged in the incision 42 to prevent the incision 42 from healing and to ensure ventilation of the middle ear 14. The ventilation tube 44 temporarily takes the place of the Eustachian tube 26 in equalizing the pressure in the middle ear 14. The ventilation tube 44 usually remains in place for three to nine months during which time the Eustachian tube 26 blockage subsides. When the tube 44 dislodges, the eardrum 22 heals; the Eustachian tube 26 then resumes its normal pressure equalizing function.

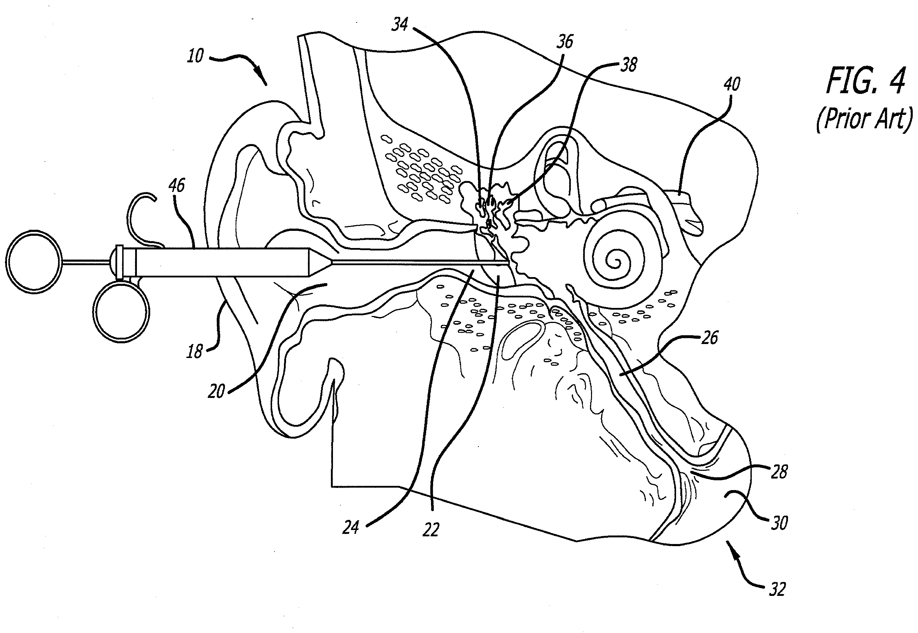

Another method of relieving the pressure in the middle ear 14 is shown in Fig. 4 in which a hypodermic needle 46 is driven through the eardrum 22 through which any accumulated fluid can be withdrawn from typically only the upper portion of the Eustachian tube 26.

The methods of Figs. 3 and 4 involve rupturing the eardrum 22 to relieve the fluid accumulation and pressure increase in the middle ear. Neither of these methods, in addition to the sometimes permanent puncture created in the eardrum 22, is especially effective in removing all of the fluid in the Eustachian tube 26 since often the lower end 28 thereof is blocked and dammed with fluid.

In connection with the above surgical treatments of Figs. 3 and 4, Eustachian tube 26 inflation is also employed to relieve the pressure build-up and fluid accumulation as shown in Fig. 5. The hypodermic syringe 46 (shown with a flexible tip 48) is inserted into a nostril or into the mouth until the tip 48 is positioned adjacent the distal opening 28 of the Eustachian tube 26 in the nasopharynx region 30 of the throat 32. Air is blown through the tip 48 via the syringe 46 into the obstructed Eustachian tube 26 and, thus, into the middle ear 14 to help relieve the congestion and reestablish middle ear ventilation. This procedure is often referred to as politzerization. Politzerization is most effective when one of the nostrils is pinched shut (as shown in Fig. 6), while the patient simultaneously swallows. This forces air into the Eustachian tube 26 and the middle ear 14. This technique is good for opening the Eustachian tube 26 but it does not clear accumulated fluid away.

Another method for clearing the middle ear 14 (at least temporarily) is referred to as the "valsalva" maneuver, accomplished by forcibly blowing air into the middle ear 14 while holding the nose, often called popping the ear. This method is also good for opening the Eustachian tube 26 but it does not clear the accumulated fluid away either.

Typical disorders associated with the middle ear and the Eustachian tube include perforated ear drums, tympanosclerosis, incus erosion, otitis media, cholesteotoma, mastoiditis, patulous Eustachian tube, and conductive hearing loss. To treat some of these disorders, ear surgery may be performed. Most ear surgery is microsurgery, performed with an operating microscope. Types of ear surgery include stapedectomy, tympanoplasty, myringotomy and ear tube surgery.

One of the simplest ear surgeries is the myringotomy or the incision of the ear drum. However, ear surgery can also require the removal of the tympanic membrane for the visualization of the middle ear space. Often surgeons will try to preserve the integrity of the membrane by making incisions in the skin of the ear canal and removing the tympanic membrane as a complete unit. Alternatively, middle ear access is achieved via the mastoids. This method approaches the middle ear space from behind the ear and drills through the mastoid air cells to the middle ear. Whether the bony partition between the external ear canal and the mastoid is removed or not depends on the extent of the disease. Canal-wall-down refers to the removal of this bony partition. Canal-wall-up refers to keeping this bony partition intact. The term modified radical mastoidectomy refers to an operation where this bony partition is removed and the eardrum and ossicles are reconstructed. A radical mastoidectomy is an operation where this bony partition is removed and the ear drum, malleus and incus bones are permanently removed so that the inner lining of the large cholesteotoma sac can be safely cleaned. This operation is done when an extensive cholesteotoma is encountered or one that is adherent to the inner ear or facial nerve. WO2006078884 discloses a guide catheter, a guidewire and a dilation catheter that can be used for dilating a Eustachian tube.

Afflictions of the middle ear and Eustachian tube are very prevalent and a serious medical problem, afflicting millions of people and causing pain, discomfort and even hearing loss or permanent ear damage. Although a number of treatments have been developed, as described above each of them has shortcomings. Therefore, a need exists for improved methods and systems for accessing, diagnosing and treating target tissue regions within the middle ear and the Eustachian tube. Ideally, such methods and systems would be minimally invasive and pose very little risk of damage to healthy ear tissue.

### SUMMARY OF THE INVENTION

The embodiments of the present invention are directed toward systems for dilating a Eustachian tube as defined in the appended claims. Various methods for accessing or treating a Eustachian tube of a patient are provided for information only.

The present disclosure provides a method for accessing a Eustachian tube of a patient using a system according to the present invention. The method may involve inserting a guide catheter into a nasal passage of the patient, the guide catheter having a distal tip with a bend having an angle between 30 and 90 degrees, and advancing the guide catheter in the nasal passage toward an opening of the Eustachian tube in the nasopharynx to place the distal tip adjacent the Eustachian tube opening.

The method may also include advancing a diagnostic device through the guide catheter to place a distal tip of the diagnostic device adjacent the Eustachian tube opening. The diagnostic device may be a catheter or an endoscope.

The method may involve introducing a diagnostic probe into the Eustachian tube to directly assess Eustachian tube function. The diagnostic probe may be made from a flexible and Eustachian tube compatible material. The diagnostic probe may be a pressure transducer located on a guidewire. The method may also include monitoring pressure within the Eustachian tube while the patient is swallowing, and assessing an opening function of the patient's Eustachian tube using the monitoring.

The method may also involve removing the guide catheter after the diagnostic probe is placed into the Eustachian tube.

The diagnostic probe may include an ultrasound probe.

The method may also involve advancing a treatment device through the guide catheter toward the Eustachian tube to place a distal tip of the treatment device adjacent the Eustachian tube opening. The treatment device may comprise a distal radiopaque member. The treatment device may comprise a catheter. The treatment device may also comprise a fluid introduction device for introducing a fluid into a middle ear space of the patient's ear. The method may also involve scanning the middle ear space using an ultrasound device. The fluid may be air, a contrast medium, an aspiration fluid, or a drug.

The treatment device may comprise an aspiration device for aspirating a substance from the middle ear space.

The method may also involve introducing a protective device proximal the Eustachian tube, and monitoring advancement of the treatment device using the protective device. In one aspect, the protective device may comprise a sensor positioned proximal the tympanic membrane to sense the position of the treatment device during the advancement. The protective device may comprise an endoscope to visualize the advancement.

The present disclosure provides a method for indirectly assessing Eustachian tube function in a patient. The method may involve positioning an energy emitter in the nasopharynx adjacent a Eustachian tube; positioning an energy receiver adjacent the tympanic membrane via the external ear canal; directing energy from the emitter toward the receiver; generating an emitter signal representative of the energy from the emitter; generating a receiver signal representative of the energy received by the emitter; forming a comparison between the emitter signal and the receiver signal; and indirectly assessing function of the Eustachian tube during swallowing, using the comparison.

The indirect assessing may involve estimating the physical characteristics of the Eustachian tube.

The energy emitter may emit energy in the form of a pressure wave or electromagnetic energy.

The present disclosure provides a method for treating a Eustachian tube in a patient. The method may involve placing a guidewire into a Eustachian tube of the patient via the patient's nasopharynx; introducing a debulking device along the guidewire into the Eustachian tube of the patient; and removing edematous tissue including hypertropic mucosa from a surface along one side of the Eustachian tube.

The guidewire may include markings and the method may also involve providing feedback related to the introducing into the Eustachian tube.

The present disclosure provides another method for treating a Eustachian tube in a patient. The method may involve introducing via the patient's nasopharynx a guidewire submucosally between cartilage and a mucosal surface of a Eustachian tube; introducing a debulking device along the guidewire into submucosal tissue of the Eustachian tube, between the cartilage and the mucosal surface; and removing some of the submucosal tissue.

The present disclosure provides a method for treating muscular dysfunction or an anatomical disorder of a Eustachian tube in a patient. The method may involve creating a lesion in at least one of a tensor villi palatine muscle or a levator villi palatine muscle to affect a stiffening of the muscle(s) upon resorption of the lesion.

The stiffening may include a shortening or a tensioning of the tensor villi palatine or the levator villi palatine.

The creating of a lesion may involve applying a therapy from the group including mechanical, laser, radio frequency and chemical therapies.

The present disclosure provides another method for treating a Eustachian tube in a patient. The method may involve placing a dual lumen pressure equalization tube through the tympanic membrane of the patient, the tube having a distal extension for location in a region of the Eustachian tube; providing a medication to the region of the Eustachian tube through a first lumen of the dual lumen tube in fluid communication with the distal extension; and providing ventilation across the tympanic membrane through a second lumen of the dual lumen tube.

The medication may be configured to reduce edema in the Eustachian tube region. The medication can include a surfactant configured to modify a surface tension of a mucosal layer of the Eustachian tube to effect an enhanced wetting of the mucosal surface with the medication.

The medication may include particles configured for capturing by mucosal tissue of the Eustachian tube to effect an extended release of the medication.

The present disclosure also provides an apparatus for treating a Eustachian tube in a patient. The apparatus may include a dual lumen tube for insertion into a tympanic membrane of the patient's ear, the tube having: a distal extension for placement in a region of the Eustachian tube; a first lumen for providing a medication to the region of the Eustachian tube through the distal extension; and a second lumen for providing ventilation across the tympanic membrane.

The first lumen may be disposed within the second lumen. The second lumen may be disposed within the first lumen. The first lumen may be disposed adjacent the second lumen.

The dual lumen tube may be made from a biodegradable bioresorbable material.

The present disclosure provides another method for treating a Eustachian tube in a patient. The method may involve accessing a Eustachian tube region via the nasopharynx, using a guide having a lumen; introducing a guidewire through the lumen of the guide to position it submucosally between cartilage and a mucosal surface of the Eustachian tube; passing a temporary intraluminal implant having a drug delivery reservoir along the guidewire to position the implant submucosally in a posterior cushion of the Eustachian tube region between the lumen and the cartilage; and delivering a drug to the Eustachian tube region from the drug delivery reservoir.

The method may also involve contemporaneously delivering a drug to adenoids and the Eustachian tube region from the drug delivery reservoir.

The drug delivery reservoir may include a coating layer disposed on the implant.

The guide may be made from a biodegradable bioresorbable material.

Yet another method for treating a Eustachian tube in a patient is disclosed. The method may involve obtaining access to a Eustachian tube region via the nasopharynx; introducing via the patient's nasopharynx a hollow guidewire dimensioned to reach into the Eustachian tube region, the hollow guidewire comprising a plurality of apertures disposed at or near its distal end; and delivering a drug to at least one of the Eustachian tube or a middle ear region of the patient's ear through the apertures.

The present disclosure provides a system for accessing a Eustachian tube of a patient. The system may include a guide configured for passing into a nasal passage of the patient to position a distal tip of the catheter at or near a Eustachian tube, the guide having distal tip with a bend having an angle between 30 and 90 degrees; and a guidewire configured to pass through the guide into the Eustachian tube.

In one aspect, the guide may include a catheter.

In another aspect, the guide may include a dual lumen tube.

In another aspect, the system may also include a diagnostic device configured for passage through the guide.

In another aspect, the system may also include a treatment device configured for passage through the guide.

The present disclosure provides a device for treating a Eustachian tube. The device may include an elongate rigid shaft. The device may also include an elongate and flexible insert coupled to the shaft, the insert including a therapeutic device for treating an elongate portion of a Eustachian tube, the insert including a lateral stiffness which deflects in accordance with the Eustachian tube, and a column stiffness which allows the insert to be pushed into the Eustachian tube without buckling.

In one aspect, the elongate rigid shaft may include a distal end with a bend ranging from 30 to 90 degrees.

In one aspect, the elongate rigid shaft may include a proximal end which may include at least one fluid fitting for supplying a fluid to the insert.

In one aspect, the elongate rigid shaft may include a lumen for passage of a guidewire.

In one aspect, the insert may include a flexible core wire.

In one aspect, the flexible core wire may be constructed from a super-elastic alloy.

In one aspect, the flexible core wire may include an atraumatic tip at a distal most portion of the insert.

In one aspect, the therapeutic device may include a balloon.

In one aspect, the balloon may include a microporous structure.

In one aspect, the balloon may be expandable to a preformed shape which matches a profile of a Eustachian tube.

In one aspect, the balloon may include a drug coating.

In one aspect, the drug coating may be one of a steroid, antibiotic, antifungal, nonsteroidal anti-inflammatory, steroidal anti-inflammatory, surfactant, or anti-mucoidal substance.

In one aspect, the therapeutic device may be detachable from the rigid shaft.

In one aspect, the therapeutic device may include a lumen.

In one aspect, the therapeutic device may be biodegradable and may include a therapeutic substance.

In one aspect, the therapeutic substance may be one of a steroid, antibiotic, antifungal, nonsteroidal anti-inflammatory, steroidal anti-inflammatory, surfactant, or anti-mucoidal substance.

In one aspect, the therapeutic device may include an expandable stent.

In one aspect, the expandable stent may include a therapeutic substance.

The present disclosure provides a method for dilating a Eustachian tube of a patient using a system of the present invention. A guide catheter may be advanced through a nasal passage of the patient to position a distal end of the guide catheter at or near an opening of the Eustachian tube of the patient. A distal portion of the guide catheter includes a bend having an angle between 30 and 90 degrees. The distal portion may be more flexible than a proximal portion of the guide catheter. A guidewire is advanced through the guide catheter such that a distal end of the guidewire enters the Eustachian tube. A dilation catheter is advanced over the guidewire to position a dilator of the dilation catheter within the Eustachian tube. The dilator is expanded to dilate the Eustachian tube. The dilation catheter and guidewire may be removed from the patient.

In one aspect, the distal portion of the guide catheter may be malleable, and a bend in the distal portion may be formed by a user of the guide catheter.

In one aspect, the opening of the Eustachian tube may include a pharyngeal ostium of the Eustachian tube, and the dilation catheter may be advanced to position the dilator in the pharyngeal ostium.

In one aspect, the guidewire may be an illuminating guidewire. Light may be emitted from the illuminating guidewire, and the emitted light may be viewed.

In one aspect, the emitted light may be viewed using an endoscope positioned in the patient's head.

In one aspect, the guide catheter may be removed from the patient before advancing the dilation catheter over the guidewire.

In one aspect, the dilation catheter may be advanced over the guidewire and through the guide catheter. The removing step may include removing the guide catheter from the patient.

In one aspect, the dilation catheter may include a balloon dilation catheter, and expanding the dilator may include inflating a balloon of the balloon dilation catheter.

In one aspect, inflating the balloon may expand a stent within the Eustachian tube.

In one aspect, the dilation catheter may include lateral wings, and expanding the dilator may include using the lateral wings to maintain the position of the balloon.

In one aspect, the balloon may be shaped when inflated to match a conical aperture of a pharyngeal ostium of the Eustachian tube ET, and expanding the dilator may include expanding the balloon within the pharyngeal ostium of the Eustachian tube ET.

In one aspect, the balloon may be shaped to have a cross-section which does not occupy the entirety of the Eustachian tube, and expanding the dilator may include maintaining the balloon in position to relieve pressure within the Eustachian tube.

In one aspect, the balloon may include cutting members, and expanding the dilator may include cutting the Eustachian tube wall with the cutting members.

In one aspect, an endoscope may be advanced through the nasal passage, and the dilation catheter may be viewed using the endoscope.

Viewing the dilation catheter includes viewing a marker on a shaft of the catheter. A location of the dilator relative to the opening of the Eustachian tube is approximated based on a distance of the marker from a proximal end of the dilator.

In one aspect, at least one substance may be applied to the Eustachian tube using the dilator.

In one aspect, the dilator may include a porous balloon for delivering the substance.

In one aspect, the dilator may include a balloon with a plurality of needles for delivering the substance.

In one aspect, the dilation catheter may apply a force against the Eustachian tube to maintain a position of the dilator during expanding.

The present disclosure provides another method for dilating a Eustachian tube of a patient. A guide catheter may be advanced through a nasal passage of the patient to position a distal end of the guide catheter at or near an opening of the Eustachian tube of the patient. A distal portion of the guide catheter may include a bend having an angle between 30 and 90 degrees. The distal portion may be more flexible than a proximal portion of the guide catheter. A delivery catheter may be advanced through the guide catheter to place the delivery catheter within the Eustachian tube. An elongate substance delivery device may be delivered into the Eustachian tube using the delivery catheter. The dilation catheter and guidewire may be removed from the patient while leaving the elongate drug delivery device in the Eustachian tube.

In one aspect, the elongate substance delivery device may be an elongate string configured to elute at least one therapeutic substance.

In one aspect, delivering the elongate substance delivery device may include internally detaching the elongate string from the delivery catheter.

In one aspect, delivering the elongate substance delivery device may include externally detaching the elongate polymer string from the delivery catheter.

In one aspect, the elongate substance delivery device may be a balloon configured to elute the substance over time.

In one aspect, delivering the elongate drug delivery device may include inflating the balloon within the Eustachian tube and decoupling the balloon from the delivery catheter.

In one aspect, the balloon may be configured to allow pressure equalization within the Eustachian tube.

In one aspect, the elongate drug delivery device may be an expandable stent.

In one aspect, delivering the elongate drug delivery device may include inserting the expandable stent into the Eustachian tube and unconstraining a proximal end of the expandable stent to allow the proximal end of the expandable stent to expand within the Eustachian tube.

In one aspect, the elongate drug delivery device may be an elongate insert including an elongate central member connected to a plurality of braces, and each brace may be connected to an elongate outer member.

In one aspect, the braces may provide and maintain open spaces in the Eustachian tube to maintain pressure equalization therein.

For a further understanding of the nature and advantages of the invention, reference should be made to the following description taken in conjunction with the accompanying figures. Each of the figures is provided for the purpose of illustration and description only and is not intended to limit the scope of the embodiments of the present invention.

### BRIEF DESCRIPTION OF THE DRAWINGS

Fig. 1 is a cross-section of a human ear showing the inner, middle and outer ear portions and the Eustachian tube connecting the middle ear with the nasopharynx region of the throat via a distal opening thereof.

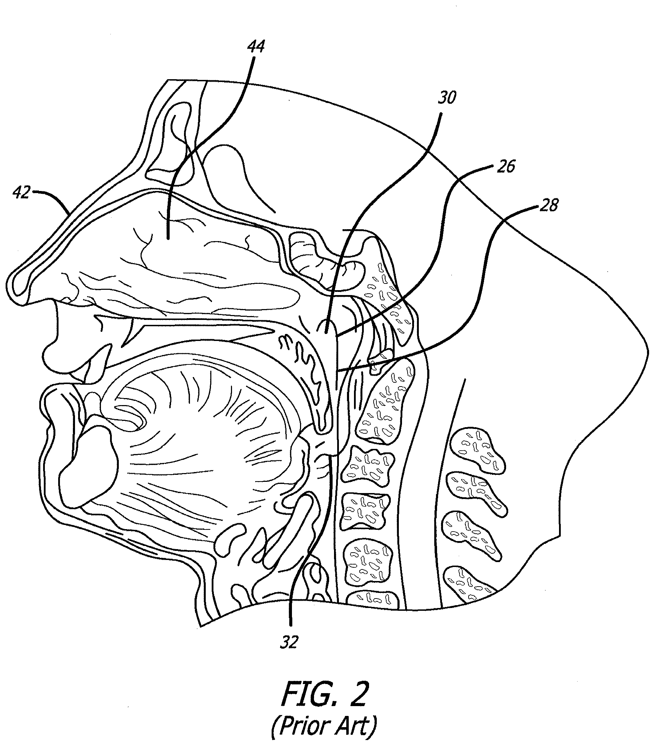

Fig. 2 is a cross-section of a human head showing the nasopharynx region of the throat illustrated in Fig. 1 containing the distal opening of the Eustachian tube illustrated in Fig. 1.
Fig. 3 is a cross-section of a human ear in the orientation shown in Fig. 1 showing a prior art surgical method for relieving fluid in the middle ear in which a ventilation tube is placed within an incision in the eardrum.
Fig. 4 is a cross-section of a human ear in the orientation shown in Fig. 1 showing a prior art surgical method for relieving fluid in the middle ear in which a syringe is shown having a needle perforating the eardrum.

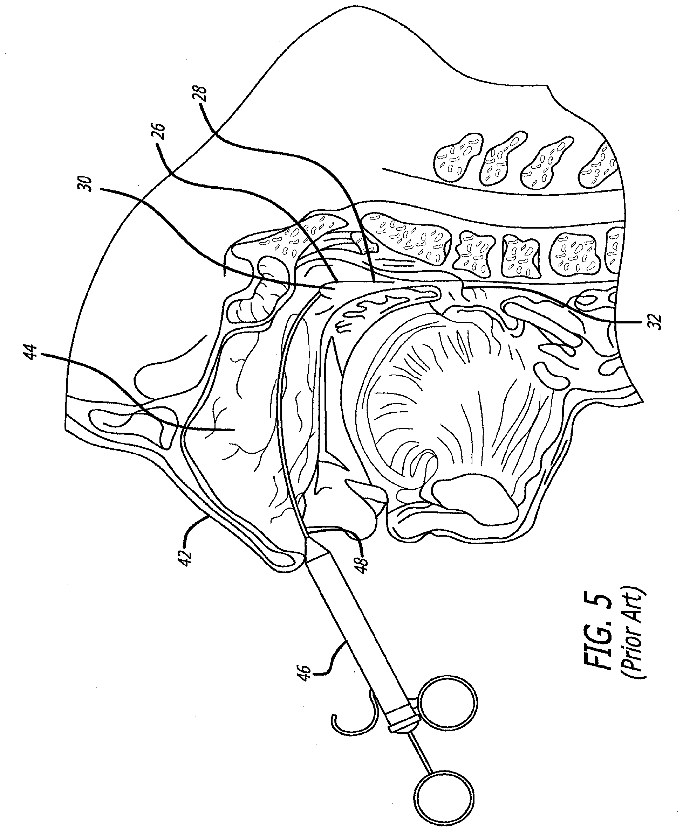

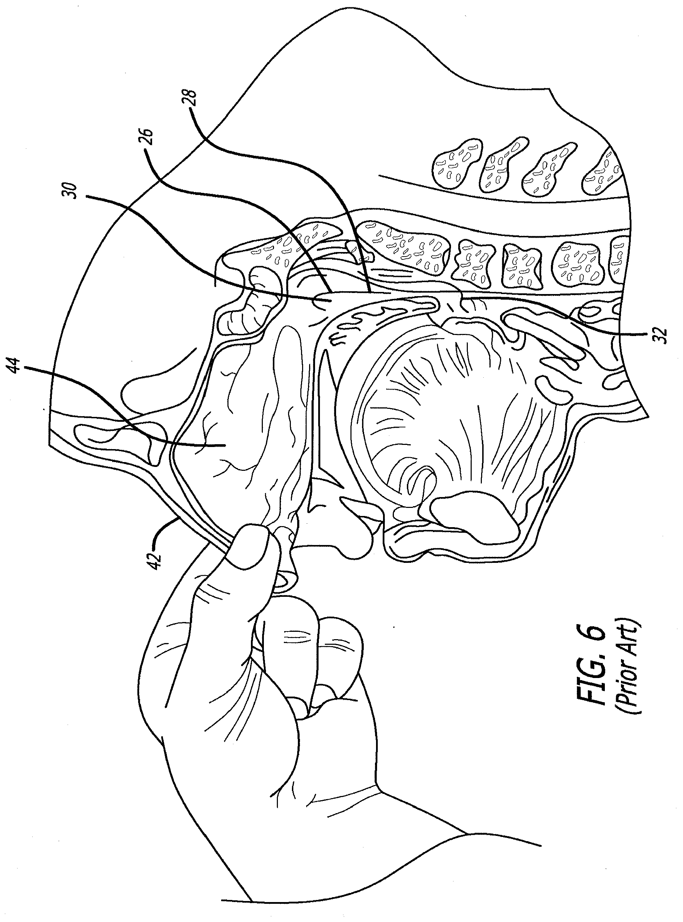

Figs. 5-6 show a cross-section of a human head in the orientation shown in Fig. 2 showing a prior art politzeration method for relieving fluid in the middle ear in which a syringe is shown having a flexible tip extending into the nose and/or throat area so that the tip abuts the distal opening of the Eustachian tube while the nose is plugged.

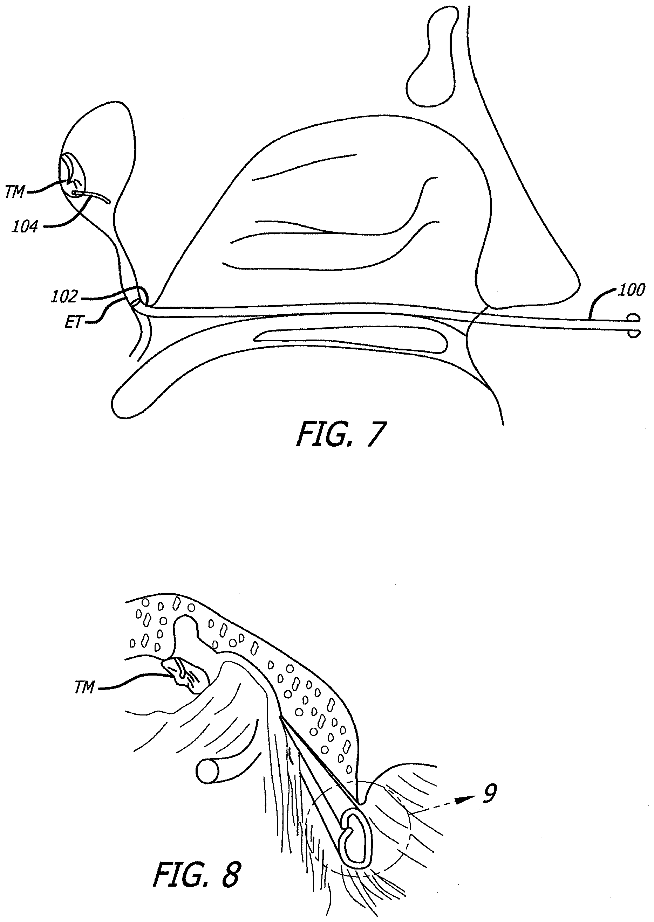

Fig. 7 shows a cross-sectional view of a human head showing the nasopharynx region and a guide catheter in the nasal passage where the distal tip of the guide catheter is adjacent the Eustachian tube opening.
Fig. 8 shows a section of the anatomical region around a Eustachian tube (ET).

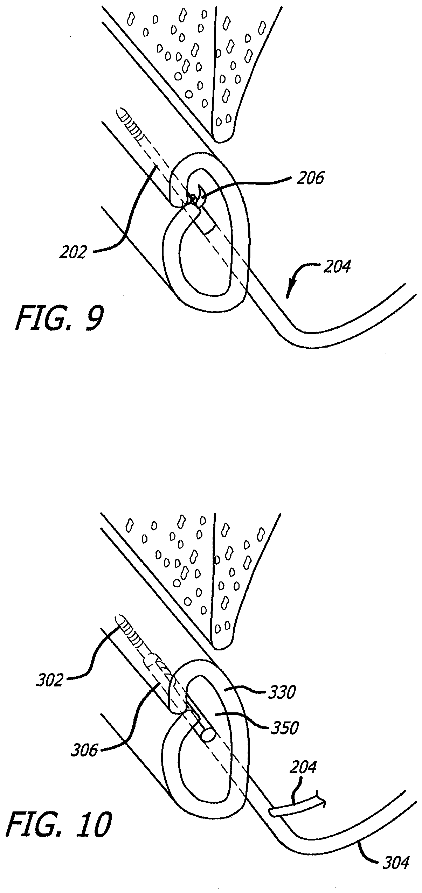

Fig. 9 shows a section of the anatomical region around a Eustachian tube showing a diagnostic or therapeutic procedure to debulk edematous tissue around the ET.
Fig. 10 shows a section of the anatomical region around a Eustachian tube showing an alternative therapeutic procedure to debulk edematous tissue around the ET.

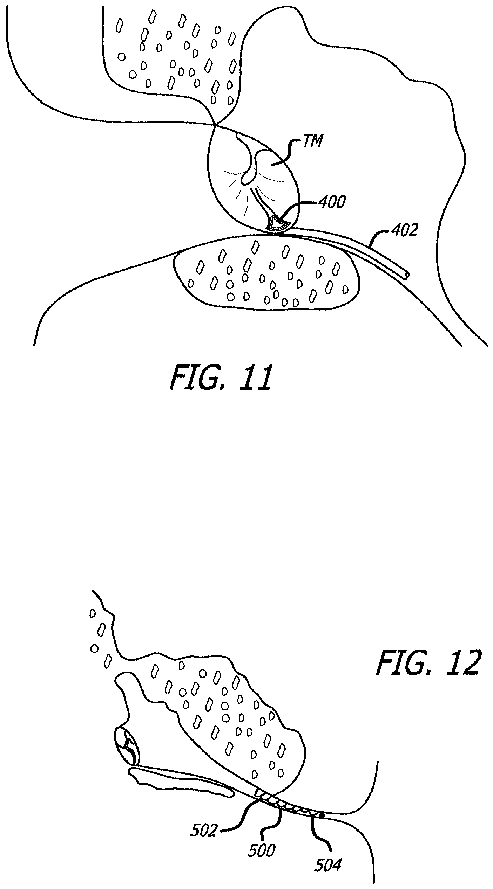

Fig. 11 shows an exemplary drug delivery system for delivering a pharmaceutical agent to treat ET inflammation or edema.
Fig. 12 shows an alternative drug delivery system for delivering a pharmaceutical agent to treat ET inflammation or edema that may be provided through the nasopharynx.

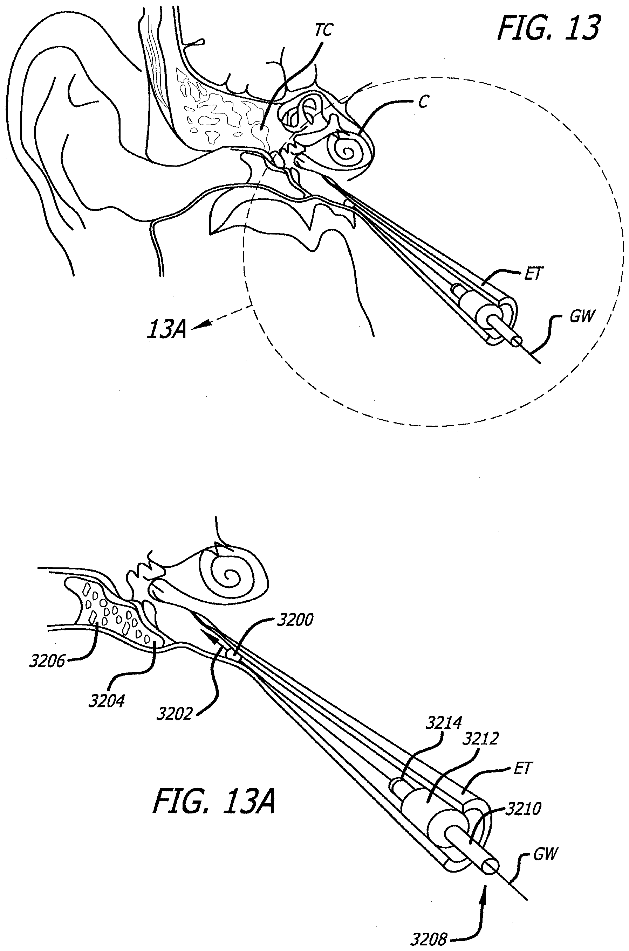

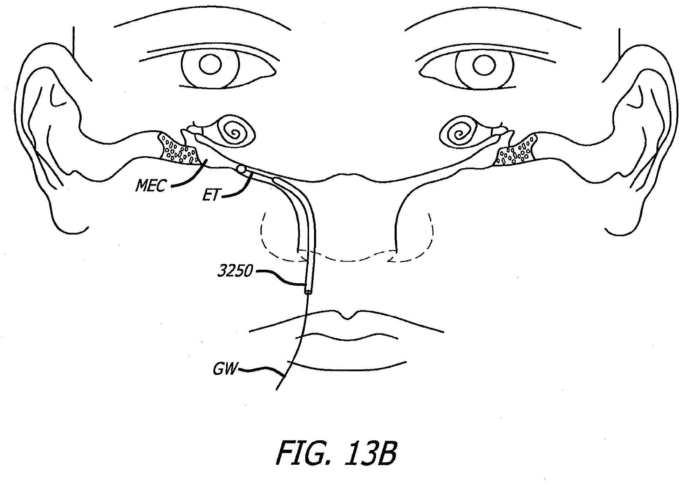

Fig. 13 shows a section of the anatomical region around the ET showing a diagnostic or therapeutic procedure being performed by devices inserted through the pharyngeal ostium of the Eustachian tube.
Fig. 13A shows an enlarged view of region 33A in Fig. 13.
Fig. 13B shows a front view of a human head with a portion of the face removed to show an embodiment of a method of introducing a guidewire into a Eustachian tube.

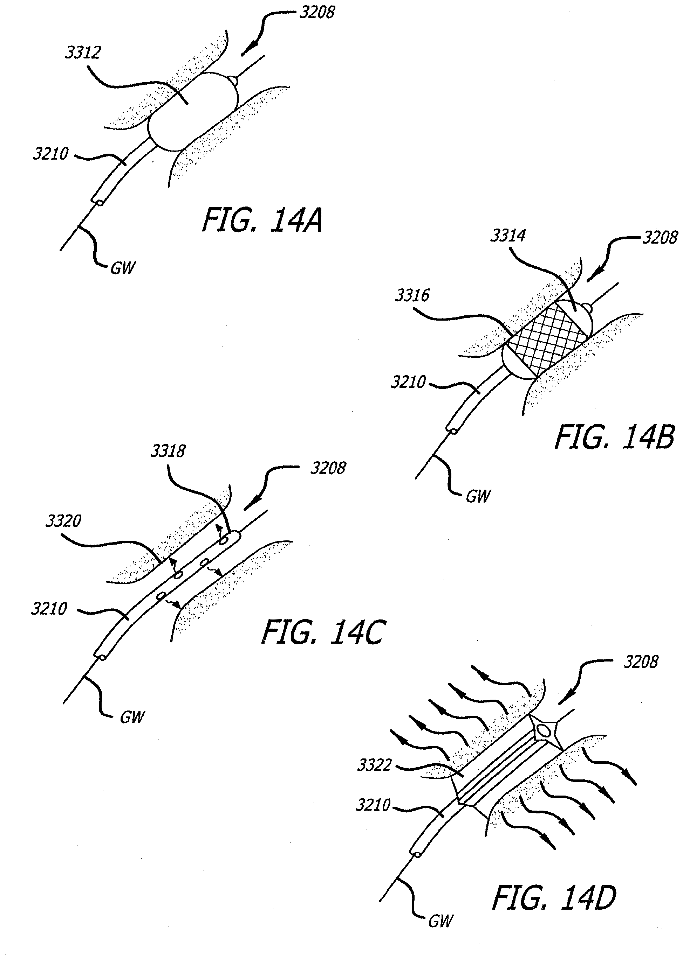

Figs. 14A - 14D illustrate various examples of working elements that could be located on the diagnostic or therapeutic device in Fig. 13.

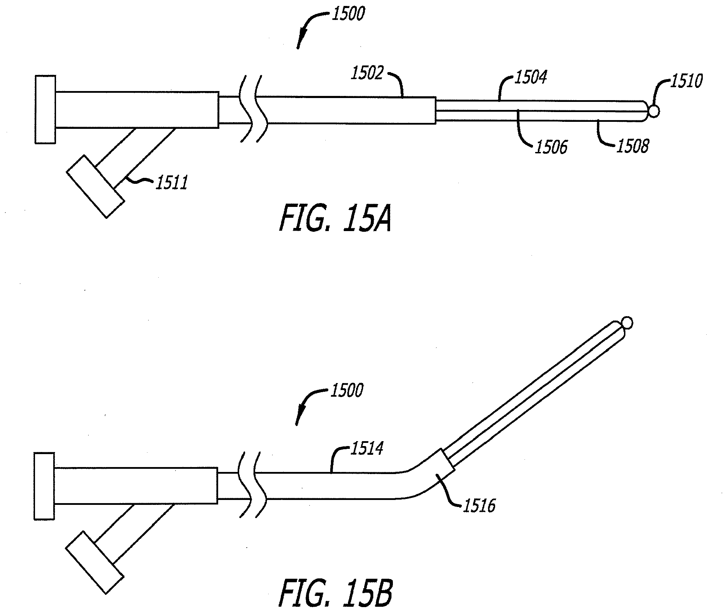

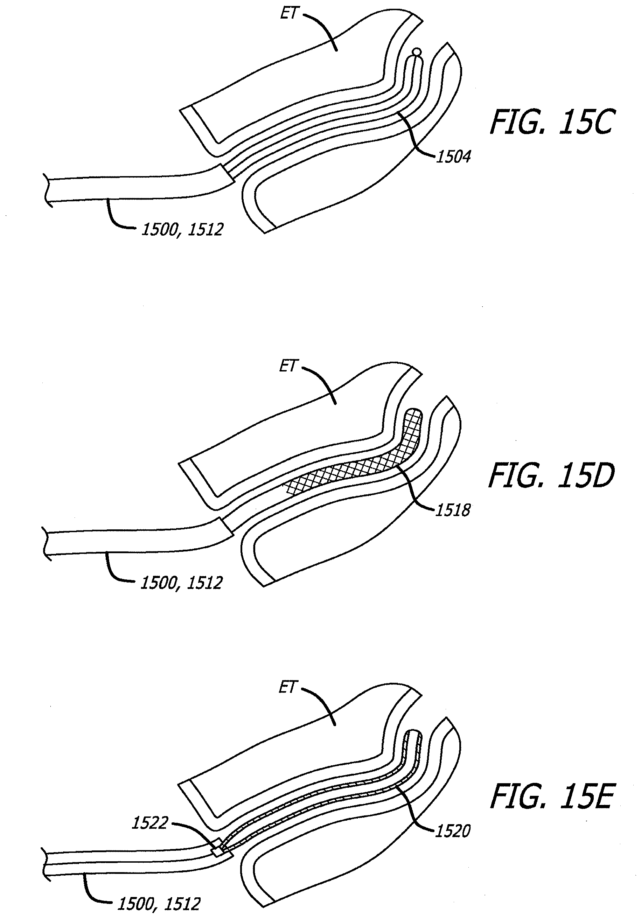

Figs. 15A and 15B show side views of example devices for providing a therapy to a Eustachian tube.
Figs. 15C - 15E show cross-sectional views of example devices providing therapies to a Eustachian tube.

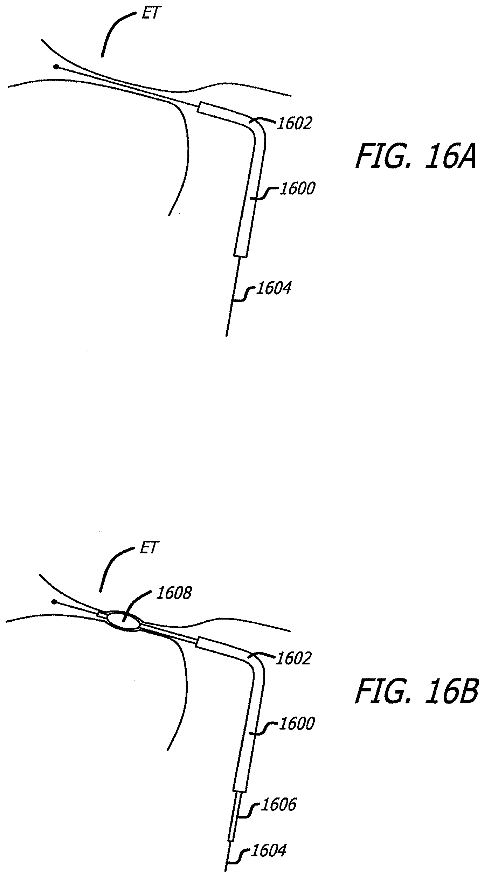

Figs. 16A and 16B show a partial cross-section of devices being used in a method for treating a Eustachian tube of a patient.

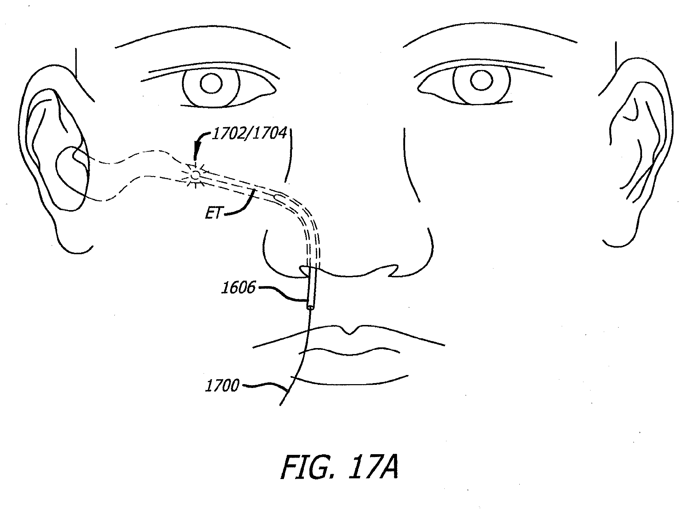

Fig. 17A shows a frontal view of an illuminated guidewire for treating a Eustachian tube in use in a patient.

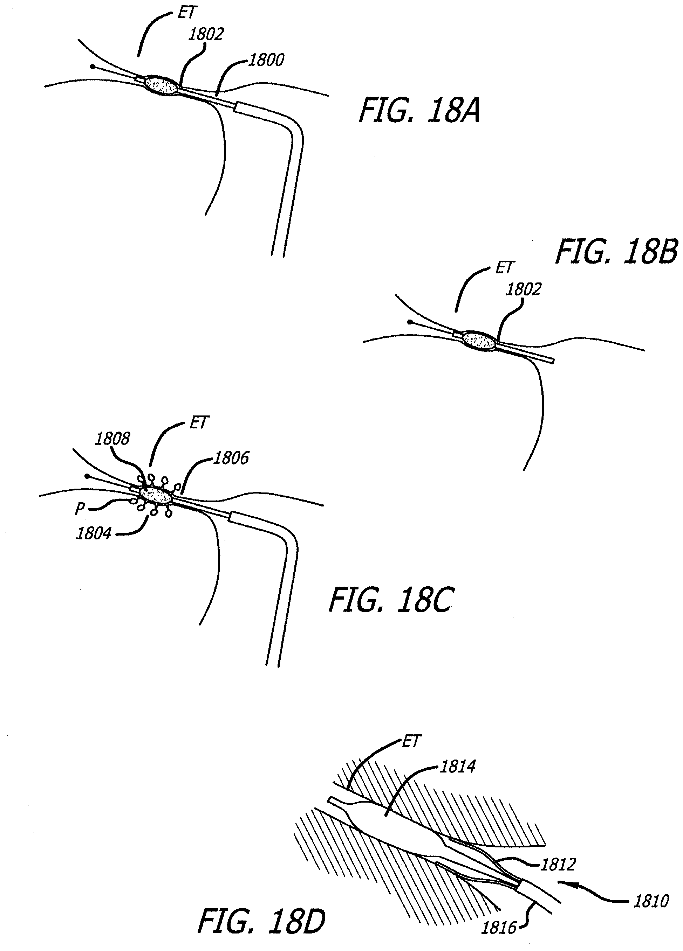

Figs. 18A and 18B show a partial cross-section of a device being used in a method for treating a Eustachian tube of a patient.
Fig. 18C shows a partial cross-section of a device being used in a method for treating a Eustachian tube of a patient.
Fig. 18D shows a partial cross-section of a device being used in a method for treating a Eustachian tube of a patient.

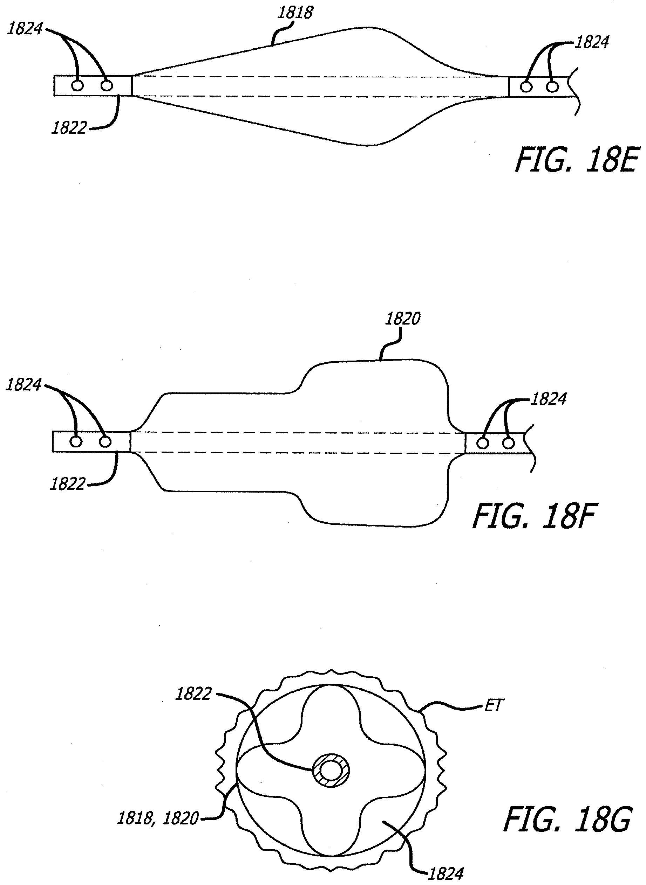

Figs. 18E and 18F show side views of a dilator for providing therapy to a Eustachian tube of a patient.
Fig. 18G shows a cross-sectional view of a dilator for providing therapy to a Eustachian tube of a patient.

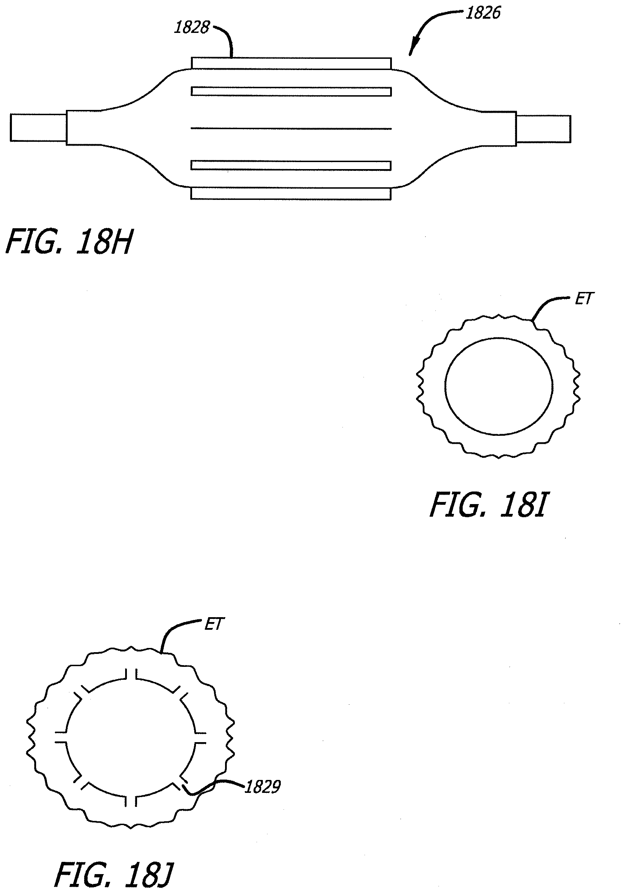

Fig. 18H shows a side view of a dilator for providing therapy to a Eustachian tube of a patient.
Figs. 18I and 18J show before and after cross-sectional views of a Eustachian tube, respectively, that was treated by the dilator of Fig. 18H.

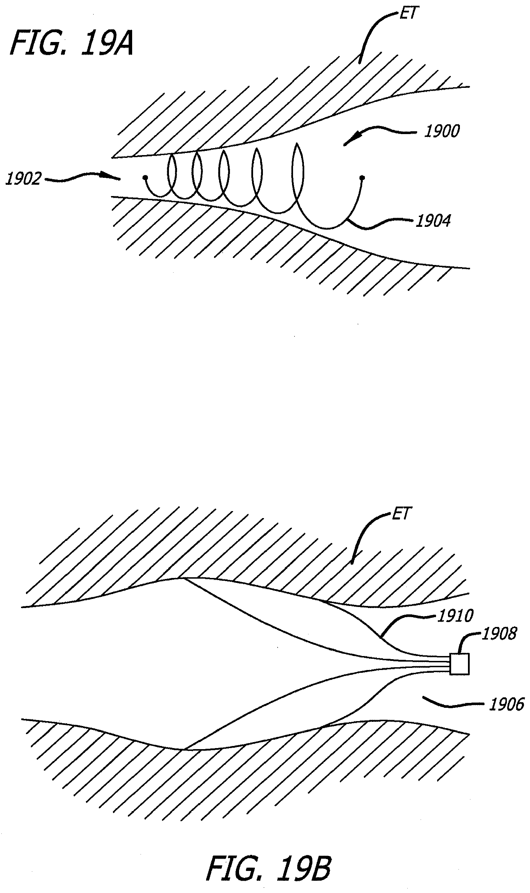

Figs. 19A and 19B show side views of a stents for providing therapy to a Eustachian tube of a patient.

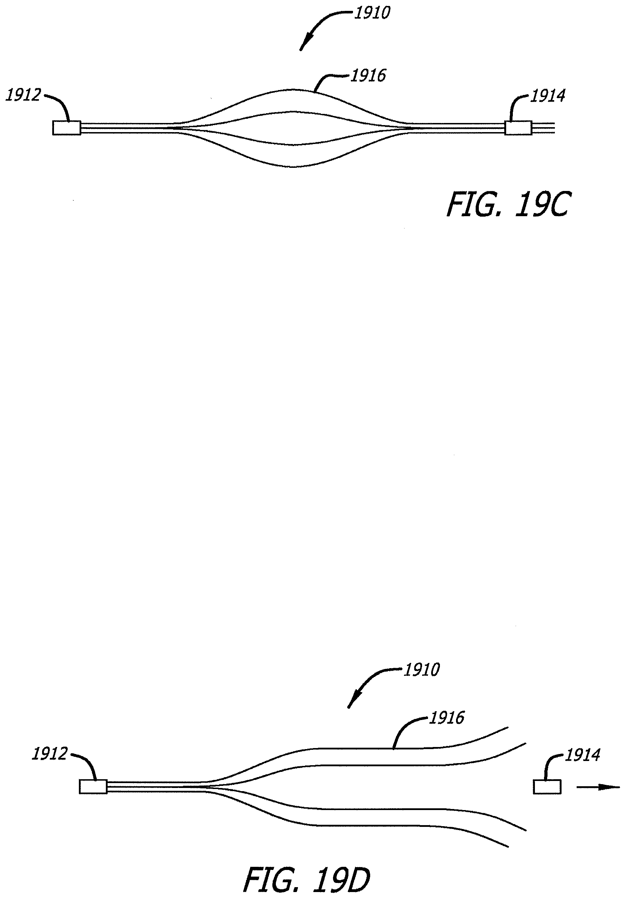

Figs. 19C and 19D show side views of a stent in different stages of expansion for providing therapy to a Eustachian tube of a patient.

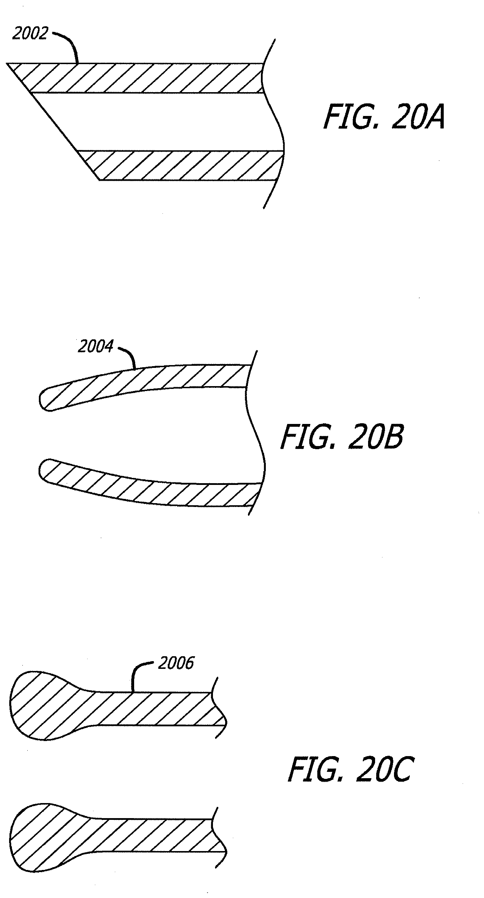

Figs. 20A, 20B and 20C show cross-sectional views of distal tips of guide catheters for interfacing with the opening of a Eustachian tube of a patient.

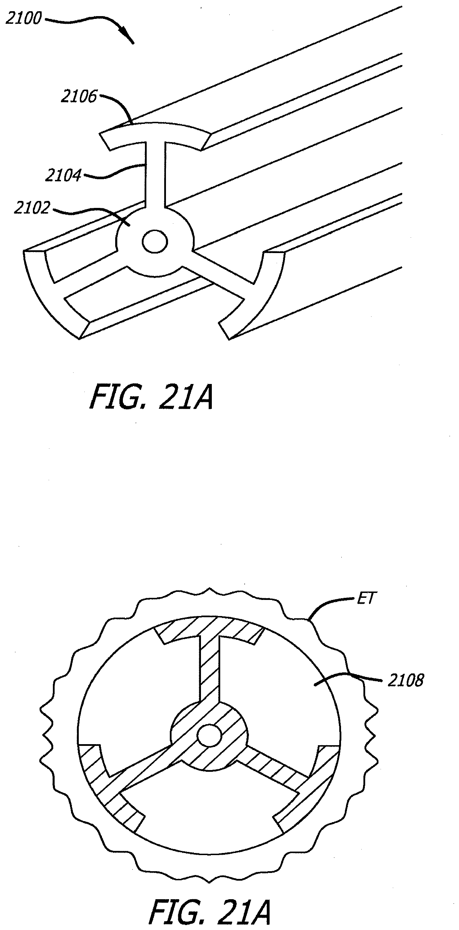

Figs. 21A and 21B show perspective and cross-sectional views, respectively, of an elongate insert for providing therapy to a Eustachian tube of a patient.

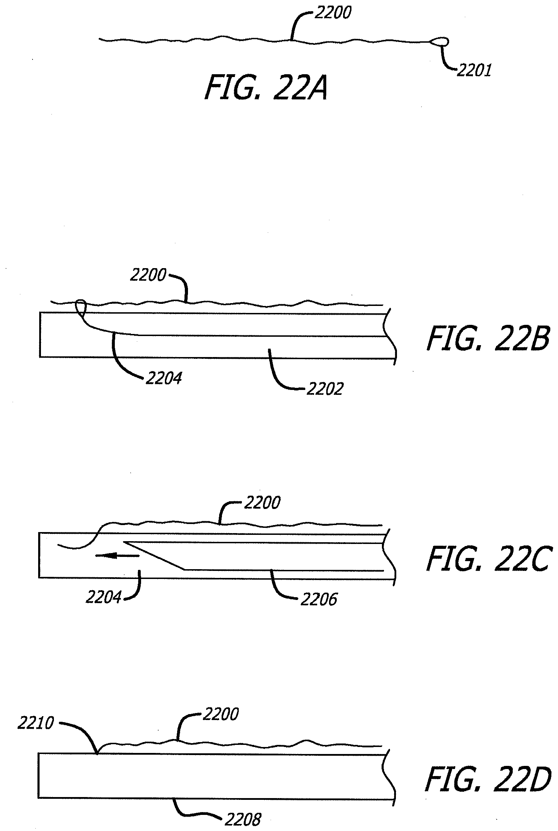

Fig. 22A shows a side view of a string insert for providing therapy to a Eustachian tube of a patient.
Figs. 22B, 22C and 22D show partial cross-sectional views of delivery catheters for delivering the string insert of Fig. 22A.

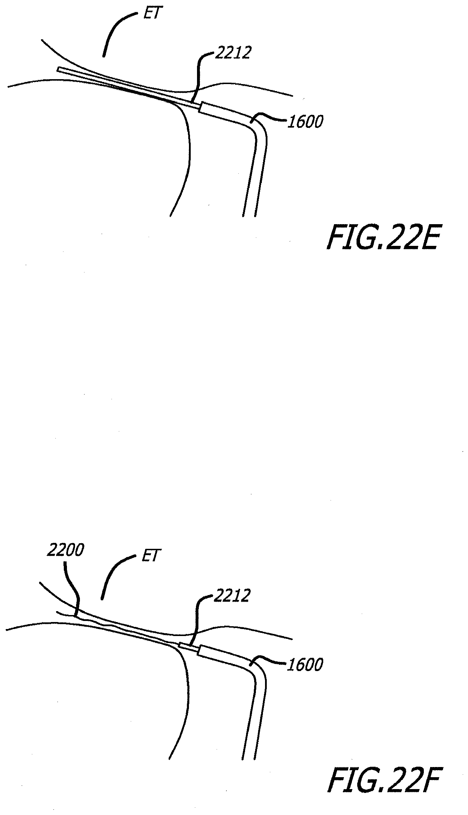

Figs. 22E and 22F show partial cross-sectional views of the string insert of Fig. 22A being used in a method for providing therapy to a Eustachian tube of a patient.

### DETAILED DESCRIPTION OF THE INVENTION

The embodiments of the present invention are directed toward systems for accessing, diagnosing and treating target tissue regions within the middle ear and the Eustachian tube.

### Access

One embodiment of the present invention is directed toward using minimally invasive techniques to gain trans-Eustachian tube access to the middle ear. In one embodiment, a middle ear space may be accessed via a Eustachian tube (ET). To obtain this access to the Eustachian tube orifice, a guide catheter having a bend on its distal tip greater than about 30 degrees and less than about 90 degrees may be used. Once accessed, diagnostic or interventional devices may be introduced into the Eustachian tube. Optionally, to prevent damage to the delicate middle ear structures, a safety mechanism may be employed. In one embodiment, the safety mechanism may include a probe and/or a sensor introduced into the middle ear via the tympanic membrane as shown in Fig. 7. For example, the probe may be an endoscope, and the sensor may be an electromagnetic transducer.

Fig. 7 is a cross-sectional view showing the nasopharynx region and a guide catheter 100 in the nasal passage where the distal tip 102 of the guide catheter is adjacent the Eustachian tube opening. Fig. 7 shows the guide catheter 100 having a bend on its distal tip 102 that is greater than about 30 degrees and less than about 90 degrees located adjacent the Eustachian tube orifice. A sensor 104 located adjacent the tympanic membrane may be used to monitor advancement of the guide catheter. The sensor is one example of a safety mechanism.

### Diagnosis

Another aspect of the present disclosure relates to diagnosis of the condition of the middle ear and its structure. Diagnosis may include use of an endoscope that has been advanced into position through the guide catheter 100. The design of the endoscope will allow for a 90 degree or more Y axis visualization and a 360 degree rotation. Such an endoscope may be used for assessment of cholesteotomas, ossicle function and/or condition, and the surgical follow-up. An exemplary endoscope that may be adapted as described above may use the IntroSpicio 115 1.8 mm camera developed by Medigus. Such a camera measures approximately 1.8 mm x 1.8 mm and its small rigid portion allows for the maximum flexibility at the endoscope tip.

Alternatively, ultrasound may be used by injecting a fluid into the middle ear space and the ET and scanning the middle ear and the ET and its structure ultrasonically. Post-procedure the fluid may be aspirated or left to drain through the Eustachian tube. An ultrasound tipped catheter may be advanced up the ET to a position at the middle ear cavity. The ultrasound catheter may then be pulled down the ET and the physician may use an external video monitor to view the structure in and adjacent the ET.

Functional diagnosis of the Eustachian tube may be achieved via direct or indirect assessment. In one embodiment, for direct assessment, the diagnostic system may allow for the dynamic monitoring of the Eustachian tube during swallowing via a diagnostic probe inserted via the nasopharynx. Since such a diagnostic system may be used dynamically during swallowing, the probe may be made of a flexible and durable material configured to be atraumatic. In one embodiment, the guide catheter(s) 100 used in the nasopharynx approach may be removed once the diagnostic probe is in or near the ET region and prior to the swallowing.

In one embodiment, the diagnostic probe may comprise an endoscope to visualize the ET structure and function. Alternatively, the diagnostic probe may include a pressure transducer located on a catheter or a wire. When a pressure transducer is used, the pressure within the ET may be monitored during swallowing and the pressure measurements may be interpreted for ET opening function. Alternatively, an ultrasound probe may be inserted in the ET lumen to scan the ET region's structure. Fluid may be introduced into the ET to facilitate ultrasound diagnosis. For any of the above diagnostic systems, a single short length transducer that is repositioned after each swallow may be used. Alternatively, an array of transducers may be used to facilitate mapping of all or a portion of an ET.

The techniques described above may be used to directly access and diagnose a Eustachian tube of a patient. A method for accessing a Eustachian tube of a patient may include inserting a guide catheter into a nasal passage of the patient, the guide catheter having a distal tip with a bend having an angle between about 30 and about 90 degrees; and advancing the guide catheter in the nasal passage toward an opening of the Eustachian tube in the nasopharynx to place the distal tip adjacent the Eustachian tube opening. Additionally, the method may also include advancing a diagnostic device through the guide catheter to place a distal tip of the diagnostic device adjacent the Eustachian tube opening. The diagnostic device may include a diagnostic catheter. The diagnostic device may include an endoscope, a pressure transducer, or an ultrasound catheter.

Additionally, the method may also include introducing a diagnostic probe into the Eustachian tube to directly assess Eustachian tube function. It is preferred that the diagnostic probe is made from a flexible and Eustachian tube compatible material. Alternatively, the diagnostic probe may comprise a pressure transducer located on a guidewire, and whereby the method also includes monitoring pressure within the Eustachian tube while the patient is swallowing; and assessing an opening function of the patient's Eustachian tube using the monitoring. The method may also include removing the guide catheter after the diagnostic probe is placed into the Eustachian tube. Additionally, or alternatively, the diagnostic probe may comprise an ultrasound probe.

For indirect functional diagnosis of a Eustachian tube, in some embodiments, an external energy source may be used to assess opening of the Eustachian tube. For example, possible energy sources may include, but are not limited to, pressure, sound, light or other electromagnetic energy. In one embodiment of indirect assessment, an emitter may be positioned in the nasopharynx and a receiver may be placed at the tympanic membrane. Correlation between the emitted signal and the received signal may be translated into the physical characteristics of the ET during swallowing.

The techniques described above may be used to implement procedures for indirectly accessing and diagnosing the Eustachian tube of a patient. The indirect assessment method includes positioning an energy emitter in the nasopharynx adjacent a Eustachian tube; positioning an energy receiver adjacent the tympanic membrane via the external ear canal; directing energy from the emitter toward the receiver; generating an emitter signal representative of the energy from the emitter; generating a receiver signal representative of the energy received by the emitter; forming a comparison between the emitter signal and the receiver signal; and indirectly assessing function of the Eustachian tube during swallowing, using the comparison. The energy emitter can be a device that emits energy in the form of a pressure wave or electromagnetic energy. The indirect assessment may also include estimating the physical characteristics of Eustachian tube.

### Treatment

Another embodiment of the present invention is directed toward the treatment of Eustachian tube disorders. In some cases, for example, Eustachian tube disorders may be related to structural obstructions of the Eustachian tube. Structural disorders of the Eustachian tube are often the result of anatomical abnormalities or excessive or edematous tissue in or around the Eustachian tube, as shown in Fig. 8. Fig. 8 shows a section of the anatomical region around a Eustachian tube ET. Fig. 8 shows some general anatomical landmarks including the tympanic membrane TM, the carotid artery, the ET cartilage as well as the location of the tensor villi palatine and the levator villi palatine muscles. Figs. 9-10 show diagnostic or therapeutic procedures being performed in the region around the ET.

Fig. 9 shows a section of the anatomical region around a Eustachian tube showing a diagnostic or therapeutic procedure to debulk edematous tissue around the ET. The procedure illustrated in Fig. 9 includes accessing the ET lumen using a guidewire 202 and removing tissue from one side of the ET using a debulking tool 204. As shown in Fig. 9, in one embodiment, the debulking tool 204 may have a retractable debulking tip 206 projecting from one side so that the tip removes tissue from one side of the ET lumen. This therapeutic procedure preferably allows for controlled access and positioning within the ET and prevents injury to opposing surfaces. It should be realized that the above-described therapeutic procedures can be performed with the aid of ultrasound guidance or visualization, for example, by using an intra-ET visualization catheter. The ultrasound can be used for diagnosis before therapy as described above. It may also be used for guidance and or assistance during the therapy.

Fig. 10 shows a section of the anatomical region around a Eustachian tube showing an alternative therapeutic procedure to debulk edematous tissue around the ET. In the alternative procedure shown in Fig. 10, the debulking device 304 may be introduced at its tip or distal end 306 submucosally between cartilage 330 and the mucosal surface, so that the mucosal surface is preserved. For this alternative procedure, the guidewire 302 and/or the debulking device may be tracked between the lumen and the cartilage, thereby protecting both the mucosal surface and the carotid artery. As shown in Fig. 10, the guidewire 302 may be inserted at a submucosal entry point between the ET cartilage and the mucosal surface. Subsequently, the debulking tool 304 may be introduced along the guidewire 302 to debulk the tissue region without affecting the mucosal surface. Ultrasound, like low power, high efficiency ultrasounds, can be used as the debulking tool to ablate, shrink or debulk tissues under the mucosal tissue.

The treatment techniques described above may be used to treat the Eustachian tube of a patient by placing a guidewire into a Eustachian tube of the patient via the patient's nasopharynx; introducing a debulking device along the guidewire into the Eustachian tube of the patient; and removing edematous tissue including hypertropic mucosa from a surface along one side of the Eustachian tube. The guidewire may include markings for providing feedback related to the introducing into the Eustachian tube. Alternatively, the debulking tool can be introduced into the ET without first placing a guidewire therein.

Alternatively, a method for treating a Eustachian tube in a patient may include introducing via the patient's nasopharynx a guidewire submucosally between cartilage and a mucosal surface of a Eustachian tube; introducing a debulking device along the guidewire into submucosal tissue of the Eustachian tube, between the cartilage and the mucosal surface; and removing some of the submucosal tissue.

In addition to the therapeutic procedures described above and illustrated in Figs. 9-10, tissue removal or remodeling (e.g. shrinkage) may be accomplished using mechanical, laser, radio frequency, and/or chemical therapies. For example, in cases where muscular dysfunction or anatomical disorder is a contributing factor, the muscles (tensor villi palatine or levator villi palatine) may be shortened or tensioned. One method of accomplishing or shortening the muscles is to create a lesion in the muscles. Over time the lesion is absorbed and the muscle tightens due to the resorbed muscular mass in a manner similar to somnoplasty.

Another embodiment of the present invention is directed toward the treatment of Eustachian tube disorders caused by inflammation or edema. In addition to the surgical procedures described above, edema may also be reduced through pharmaceutical therapy. Delivery of therapeutic agents, especially steroids, into the ET mucosa may be facilitated locally using a range of methods including aspirating directly into the ET using a micro-catheter designed to enter either the nasopharynx or the middle ear side of the ET. Alternatively, an agent may be delivered from the surface of a dilation balloon. In this case, the agent may be deposited into the mucosal layer rather than onto its surface. Sustained delivery may be facilitated by depositing the drug into a reservoir and embedding the reservoir into the mucosa. Extending the residence time of therapeutic agents may be achieved by including the agents as particles and charging the reservoir particles such that they adhere to the mucosa surface. Alternatively, the residence time of therapeutic agents may be controlled by implanting the reservoir into the ET or its substructure.

An exemplary drug delivery system according to one embodiment is shown in Fig. 11. As shown in Fig. 11, a pressure equalization tube 400 may be inserted into the tympanic membrane. The pressure equalization tube includes an extension 402 that resides in the region of the Eustachian tube, where the extension has drug delivery capabilities. As shown on Fig. 11, the pressure equalization tube 400 may be dual-lumen to provide drug delivery and ventilation functions. The pressure equalization tube 400 having an extension 402 may be designed to slide between the radial fibers of the tympanic membrane TM. When in place the tube may be oriented to minimize migration paths.

Alternatively, a drug delivery system may be provided through the nasopharynx as illustrated in Fig. 12. As shown in Fig. 12, the drug delivery may be provided from an intraluminal temporary implant 500. The temporary nature of the implant 500 may require a removal system or may provide for natural removal through degradation and/or digestion. Similar to the debulking devices described above, the drug delivery system may also be implanted submucosally, thus having the benefit of not obstructing the surface mucosa. In one embodiment, the implant may be deployed into the posterior cushion of the ET between the lumen and the cartilage. This method may benefit from the use of consistent anatomical landmarks and may minimize the likelihood of trauma to the middle ear or carotid artery. The implant 500 may include an anchored drug delivery reservoir in the form of a coil having a reducing diameter distal 502 to proximal 504, respectively.

Fig. 13 shows a section of the anatomical region around a Eustachian tube ET showing a diagnostic or therapeutic procedure being performed by devices inserted through the pharyngeal ostium of the Eustachian tube. Fig. 13 shows a guidewire GW inserted into a desired region in the ET through the nasopharynx and a diagnostic or therapeutic procedure being performed by a device introduced into the Eustachian tube over guidewire GW.

Fig. 13A shows an enlarged view of region 13A in Fig. 13 showing the anatomical region around a Eustachian tube ET showing a diagnostic or therapeutic procedure being performed by devices inserted through the pharyngeal ostium of the Eustachian tube. In one embodiment, guidewire GW comprises an anchoring balloon 3200 located on the distal region of guidewire GW. Anchoring balloon 3200 is inflated after positioning guidewire GW at a target location. Anchoring balloon 3200 anchors guidewire GW to the adjacent anatomy and prevents accidental repositioning of guidewire GW during a diagnostic or therapeutic procedure. Anchoring balloon 3200 may be made from any suitable compliant or semi-compliant material, such as but not limited to crosslinked polyethylene or other polyolefins, polyurethane, flexible polyvinylchloride, Nylon, or the like. In various alternative embodiments, guidewire GW may include one or more anchoring elements other than anchoring balloon 3200, such as a notch on guidewire GW, a bent region on guidewire GW, a self-expanding element, a hook, a coiled element, or the like. In another embodiment, guidewire GW may include a sensor 3202 located on the distal region of guidewire GW. Sensor 3202 may enable guidewire GW to be used in conjunction with a suitable surgical navigation system. In one embodiment, sensor 3202 may include an electromagnetic sensor used in conjunction with an electromagnetic surgical navigation system such as GE lnstaTrakTM3 500 plus system. One or more sensor 3202 or other types of surgical navigation sensors or transmitters may also be located on other diagnostic or therapeutic devices disclosed herein. Sensor 3202 may be used in conjunction with a stationary sensor 3204 located in the external ear. The combination of sensor 3202 and stationary sensor 3204 may facilitate positioning of guidewire GW in a target region.

In another embodiment, a radiopaque plug 3206 may be inserted from the external ear to a region adjacent to an eardrum. Radiopaque plug 3206 may serve as a fiducial marker during preoperative scanning of the patient and thus may enable a physician to accurately position a diagnostic or therapeutic device close to the eardrum. Other image guidance methods and devices may also be used in conjunction with diagnostic or therapeutic procedures disclosed herein. Fig. 13A also shows a diagnostic or therapeutic device 3208 comprising a shaft 3210 and a working element 3212, e.g. a dilating balloon being introduced over guidewire GW. Diagnostic or therapeutic device 3208 may comprise a radiopaque marker 3214.

Fig. 13B shows a front view of a human head with a portion of the face removed to show an exemplary method of introducing a guidewire into a Eustachian tube. In Fig. 13B, a guide catheter 3250 is introduced through a nostril into the nasopharynx. A distal portion of guide catheter 3250 may comprise a bent or angled region. For example, in one embodiment such bent or angled region may form an internal angle ranging from about 45 degrees to about 150 degrees. Guide catheter 3250 may be constructed using one of the various designs disclosed in the assignee's copending patent application No. 11/926,565 (attorney docket No. ACLRT-021BC7). Guide catheter 3250 is positioned in the nasopharynx such that the distal tip of guide catheter 3250 is located near a nasopharyngeal opening of a Eustachian tube. Thereafter, a guidewire GW is introduced through guide catheter 3250 into the Eustachian tube. Guidewire GW can then be used to advance one or more diagnostic or therapeutic devices into the Eustachian tube to perform one or more diagnostic or therapeutic procedures.

Figs. 14A - 14D illustrate various embodiments of working elements that may be located on a diagnostic or therapeutic device like the one shown in Fig. 13. Fig. 14A shows an example of a working element comprising a dilating balloon. Dilating balloon 3312 may be made from a suitable non-compliant material, such as but not limited to polyethylene terephthalate, Nylon, or the like.

Fig. 14B shows an example of a working element comprising a dilating balloon 3314 loaded with a balloon-expandable stent 3316. In some embodiments, dilating balloon 3314 may be made from a suitable non-compliant material, such as but not limited to polyethylene terephthalate, Nylon, or the like. Several types of stent designs may be used to construct stent 3316, such as but not limited to metallic tube designs, polymeric tube designs, chain-linked designs, spiral designs, rolled sheet designs, single wire designs, or the like. These designs may have an open-cell or closed-cell structure. A variety of fabrication methods may be used for fabricating stent 3316, including but not limited to laser cutting a metal or polymer element, welding metal elements, etc. A variety of materials may be used for fabricating stent 3316, including but not limited to metals, polymers, foam type materials, plastically deformable materials, super elastic materials, and the like. A variety of features may be added to stent 3316, including but not limited to radiopaque coatings, drug elution mechanisms to elute anti-inflammatory agents, antibiotics, and the like. In one embodiment, stent 3316 may be bioabsorbable. Working elements may also comprise a self-expanding stent instead of a pressure-expandable stent.

Fig. 14C shows an example of a working element comprising a lavage element 3318. Lavage element 3318 may include multiple lavage openings 3320. Lavage openings 3320 may be connected to a lavage lumen in shaft 3210, through which suitable lavage media such as solutions containing contrast agents, pharmaceutically acceptable salt or dosage form of an antimicrobial agent (e.g. antibiotic, antiviral, anti-parasitic, antifungal, etc.), an anesthetic agent with or without a vasoconstriction agent (e.g. Xylocaine with or without epinephrine, Tetracaine with or without epinephrine, etc.), an analgesic agent, a corticosteroid or other anti-inflammatory (e.g. an NSAID), a decongestant (e.g. vasoconstrictor), a mucous thinning agent (e.g. an expectorant or mucolytic), an agent that prevents or modifies an allergic response (e.g. an antihistamine, cytokine inhibitor, leucotriene inhibitor, IgE inhibitor, immunomodulator), an allergen or another substance that causes secretion of mucous by tissues, hemostatic agents to stop bleeding, antiproliferative agents, cytotoxic agents (e.g. alcohol), biological agents such as protein molecules, stem cells, genes or gene therapy preparations, or the like may be delivered. In one embodiment, a fraction of lavage openings 3320 may be connected to an aspiration lumen to aspirate the lavage media out of the Eustachian tube.

Fig. 14D shows an example of a working element comprising a substance delivery reservoir 3322. Substance delivery reservoir 3322 may be fully or partially biodegradable or non-biodegradable. In one embodiment, substance delivery reservoir 3322 is made of a suitable biocompatible material such as hydrogel (e.g. collage hydrogel). In another embodiment, substance delivery reservoir 3322 comprises a porous matrix formed of a porous material such as a flexible or rigid polymer foam, cotton wadding, gauze, etc. Examples of biodegradable polymers that may be foamed or otherwise rendered porous include polyglycolide, poly-L-lactide, poly-Dlactide, poly(amino acids), polydioxanone, polycaprolactone, polygluconate, polylactic acid-polyethylene oxide copolymers, modified cellulose, collagen, polyorlhoesters, polyhydroxybutyrate, polyanhydride, polyphosphoester, poly(alpha-hydroxy acid) and combinations thereof. Examples of nonbiodegradable polymers that may be foamed or otherwise rendered porous include polyurethane, polycarbonate, silicone elastomers, etc. Substance delivery reservoir 3322 may also include one or more embodiments disclosed in U.S. Patent Application No. 10/912,578 entitled "Implantable Device and Methods for Delivering Drugs and Other Substances to Treat Sinusitis and Other Disorders" filed on August 4, 2004. The substance delivery reservoir 3322 or any substance delivery devices described in this application may be used to deliver various types of therapeutic or diagnostic agents. The term "diagnostic or therapeutic substance" as used herein is to be broadly construed to include any feasible drugs, prodrugs, proteins, gene therapy preparations, cells, diagnostic agents, contrast or imaging agents, biologicals, etc. Such substances may be in bound or free form, liquid or solid, colloid or other suspension, solution or may be in the form of a gas or other fluid or non-fluid. For example, in some applications where it is desired to treat or prevent a microbial infection, the substance delivered may comprise pharmaceutically acceptable salt or dosage form of an antimicrobial agent (e.g. antibiotic, antiviral, antiparacytic, antifungal, etc.), a corticosteroid or other anti-inflammatory (e.g. an NSAID), a decongestant (e.g. vasoconstrictor), a mucous thinning agent (e.g. an expectorant or mucolytic), an agent that prevents or modifies an allergic response (e.g. an antihistamine, cytokine inhibitor, leucotriene inhibitor, IgE inhibitor), etc.

Some nonlimiting examples of antimicrobial agents that may be used include acyclovir, amantadine, aminoglycosides (e.g. amikacin, gentamicin and tobramycin), amoxicillin, amoxicillinlclavulanate, amphotericin B, ampicillin, ampicillinlsulbactam, atovaquone, azithromycin, cefazolin, cefepime, cefotaxime, cefotetan, cefpodoxime, ceflazidime, ceflizoxime, ceftriaxone, cefuroxime, cefuroxime axetil, cephalexin, chloramphenicol, clotrimazole, ciprofloxacin, clarithromycin, clindamycin, dapsone, dicloxacillin, doxycycline, erythromycin, fluconazole, foscarnet, ganciclovir, atifloxacin, imipenemlcilastatin, isoniazid, itraconazole, ketoconazole, metronidazole, nafcillin, nafcillin, nystatin, penicillin, penicillin G, pentamidine, piperacillinltazobactam, rifampin, quinupristin-dalfopristin, ticarcillinlclavulanate, trimethoprimlsulfamethoxazole, valacyclovir, vancomycin, mafenide, silver sulfadiazine, mupirocin (e.g. Bactroban, Glaxo SmithKline, Research Triangle Park, North Carolina), nystatin, triarncinolonelnystatin, clotrimazolelbetamethasone, clotrimazole, ketoconazole, butoconazole, miconazole, tioconazole; detergent-like chemicals that disrupt or disable microbes (e.g. nonoxynol-9, octoxynol-9, benzalkonium chloride, menfegol, and N-docasanol); chemicals that block microbial attachment to target cells and/or inhibit entry of infectious pathogens (e.g. sulphated and sulphonated polymers such as PC-515 (carrageenan), Pro-2000, and Dextrin 2 Sulphate); antiretroviral agents (e.g. PMPA gel) that prevent retroviruses from replicating in the cells; genetically engineered or naturally occurring antibodies that combat pathogens such as anti-viral antibodies genetically engineered from plants known as "plantibodies"; agents which change the condition of the tissue to make it hostile to the pathogen (such as substances which alter mucosal pH (e.g. Buffer Gel and Acid form)); non-pathogenic or "friendly" microbes that cause the production of hydrogen peroxide or other substances that kill or inhibit the growth of pathogenic microbes (e.g. lactobacillus); antimicrobial proteins or peptides such as those described in U.S. Patent No. 6,716,813 (Lin et al.), or antimicrobial metals (e.g. colloidal silver).

Additionally or alternatively, in some applications where it is desired to treat or prevent inflammation the substances delivered may include various steroids or other anti-inflammatory agents (e.g. nonsteroidal anti-inflammatory agents or NSAIDS), analgesic agents or antipyretic agents. For example, corticosteroids that have previously administered by intranasal 10 administration may be used, such as beclomethasone (Vancenase® or Beconase), flunisolide (Nasalid®), fluticasone proprionate (Flonase®), triamcinolone acetonide (Nasacort®), budesonide (Rhinocort Aqua®), loterednol etabonate (Locort) and mometasone (Nasonex®). Other salt forms of the aforementioned corticosteroids may also be used. Also, other non-limiting examples of steroids that may be useable include but are not limited to aclometasone, desonide, hydrocortisone, betamethasone, clocortolone, desoximetasone, fluocinolone, flurandrenolide, mometasone, prednicarbate, amcinonide, desoximetasone, diflorasone, fluocinolone, fluocinonide, halcinonide, clobetasol, augmented betamethasone, diflorasone, halobetasol, prednisone, dexarnethasone and methylprednisolone. Other anti-inflammatory, analgesic or antipyretic agents that may be used include the Nonselective COX Inhibitors (e.g. salicylic acid derivatives, aspirin, sodium salicylate, choline magnesium trisalicylate, salsalate, diflunisal, sulfasalazine and olsalazine; para-aminophenol derivatives such as acetaminophen; indole and indene acetic acids such as indomethacin and sulindac; heteroaryl acetic acids such as tolmetin, dicofenac and ketorolac; arylpropionic acids such as ibuprofen, naproxen, flurbiprofen, ketoprofen, fenoprofen and oxaprozin; anthranilic acids (fenamates) such as mefenamic acid and meloxicam; enolic acids such as the oxicams (piroxicam, meloxicam) and alkanones such as nabumetone) and Selective COX-2 Inhibitors (e.g. diaryl-substituted furanones such as rofecoxib; diaryl-substituted pyrazoles such as celecoxib; indole acetic acids such as etodolac; and sulfonanilides such as nimesulide).

Additionally or alternatively, in some applications, such as those where it is desired to treat or prevent an allergic or immune response and/or cellular proliferation, the substances delivered may include a) various cytokine inhibitors such as humanized anti-cytokine antibodies, anti-cytokine receptor antibodies, recombinant (new cell resulting from genetic recombination) antagonists, or soluble receptors; b) various leucotriene modifiers such as zafirlukast, montelukast and zileuton; c) immunoglobulin E (IgE) inhibitors such as Omalizumab (an anti-lgE monoclonal antibody formerly called rhu Mab-E25) and secretory leukocyte protease inhibitor; and d) SYK Kinase inhibitors such as an agent designated as "R-112" manufactured by Rigel Pharmaceuticals, Inc., South San Francisco, California.

Additionally or alternatively, in some applications, such as those where it is desired to shrink mucosal tissue, cause decongestion, or effect hemostasis, the substances delivered may include various vasoconstrictors for decongestant and or hemostatic purposes including but not limited to pseudoephedrine, xylometazoline, oxymetazoline, phenylephrine, epinephrine, etc.

Additionally or alternatively, in some applications, such as those where it is desired to facilitate the flow of mucous, the substances delivered may include various mucolytics or other agents that modify the viscosity or consistency of mucous or mucoid secretions, including but not limited to acetylcysteine. In one particular embodiment, the substance delivered comprises a combination of an anti-inflammatory agent (e.g. a steroid or an NSAID) and a mucolytic agent.

Additionally or alternatively, in some applications such as those where it is desired to prevent or deter histamine release, the substances delivered may include various mast cell stabilizers or drugs which prevent the release of histamine such as crornolyn (e.g. Nasal Chroma) and nedocromil.

Additionally or alternatively, in some applications such as those where it is desired to prevent or inhibit the effect of histamine, the substances delivered may include various antihistamines such as azelastine (e.g. Astylin) diphenhydramine, loratidine, etc.

Additionally or alternatively, in some embodiments such as those where it is desired to dissolve, degrade, cut, break or remodel bone or cartilage, the substances delivered may include substances that weaken or modify bone and/or cartilage to facilitate other procedures wherein bone or cartilage is remodeled, reshaped, broken or removed. One example of such an agent would be a calcium chelator such as EDTA that could be injected or delivered in a substance delivery implant next to a region of bone that is to be remodeled or modified. Another example would be a preparation consisting of or containing bone degrading cells such as osteoclasts. Other examples would include various enzymes of material that may soften or break down components of bone or cartilage such as collagenase (CGN), trypsin, trypsinlEDTA, hyaluronidase, and tosyllysylchloromethane (TLCM).

Additionally or alternatively, in some applications, the substances delivered may include other classes of substances that are used to treat rhinitis, nasal polyps, nasal inflammation, and other disorders of the ear, nose and throat including but not limited to anti-cholinergic agents that tend to dry up nasal secretions such as ipratropium (Atrovent Nasal®), as well as other agents not listed here.

Additionally or alternatively, in some applications such as those where it is desired to draw fluid from polyps or edematous tissue, the substances delivered may include locally or topically acting diuretics such as furosemide and/or hyperosmolar agents such as sodium chloride gel or other salt preparations that draw water from tissue or substances that directly or indirectly change the osmolar content of the mucous to cause more water to exit the tissue to shrink the polyps directly at their site.

Additionally or alternatively, in some applications such as those wherein it is desired to treat a tumor or cancerous lesion, the substances delivered may include antitumor agents (e.g. cancer chemotherapeutic agents, biological response modifiers, vascularization inhibitors, hormone receptor blockers, cryotherapeutic agents or other agents that destroy or inhibit neoplasia or tumorigenesis) such as alkylating agents or other agents which directly kill cancer cells by attacking their DNA (e.g. cyclophosphamide, isophosphamide), nitrosoureas or other agents which kill cancer cells by inhibiting changes necessary for cellular DNA repair (e.g. carmustine (BCNU) and lomustine (CCNU)), antimetabolites and other agents that block cancer cell growth by interfering with certain cell functions, usually DNA synthesis (e.g. 6 mercaptopurine and 5-fluorouracil (5FU), antitumor antibiotics and other compounds that act by binding or intercalating DNA and preventing RNA synthesis (e.g. doxorubicin, daunorubicin, epirubicin, idarubicin, mitomycin-C and bleomycin) plant (vinca) alkaloids and other antitumor agents derived from plants (e.g. vincristine and vinblastine), steroid hormones, hormone inhibitors, hormone receptor antagonists and other agents which affect the growth of hormone-responsive cancers (e.g. tamoxifen, herceptin, aromatase inhibitors such as aminoglutetharnide and formestane, trriazole inhibitors such as letrozole and anastrazole, steroidal inhibitors such as exemestane), antiangiogenic proteins, small molecules, gene therapies and/or other agents that inhibit angiogenesis or vascularization of tumors (e.g. meth-I, meth-2, thalidomide), bevacizumab (Avastin), squalamine, endostatin, angiostatin, Angiozyme, AE-941 (Neovastat), CC-5013 (Revimid), medi-522 (Vitaxin), 2-methoxyestradiol (2ME2, Panzem), carboxyamidotriazole (CAI), combretastatin A4 prodrug (CA4P), SU6668, SU11248, BMS-275291, COL-3, EMD 121974, IMC-1C11, 1M862, TNP-470, celecoxib (Celebrex), rofecoxib (Vioxx), interferon alpha, interleukin-12 (IL-12) or any of the compounds identified in Science Vol. 289, pages 1197-1201 (August 17, 2000), which is expressly incorporated herein by reference, biological response modifiers (e.g. interferon, bacillus calmetteguerin (BCG), monoclonal antibodies, interluken 2, granulocyte colony stimulating factor (GCSF), etc.), PGDF receptor antagonists, herceptin, asparaginase, busulphan, carboplatin, cisplatin, carmustine, cchlorambucil, cytarabine, dacarbazine, etoposide, flucarbazine, flurouracil, gemcitabine, hydroxyurea, ifosphamide, irinotecan, lomustine, melphalan, mercaptopurine, methotrexate, thioguanine, thiotepa, tomudex, topotecan, treosulfan, vinblastine, vincristine, mitoazitrone, oxaliplatin, procarbazine, streptocin, taxol, taxotere, analogslcongeners and derivatives of such compounds as well as other antitumor agents not listed here.

Additionally or alternatively, in some applications such as those where it is desired to grow new cells or to modify existing cells, the substances delivered may include cells (mucosal cells, fibroblasts, stem cells or genetically engineered cells) as well as genes and gene delivery vehicles such as plasmids, adenoviral vectors or naked DNA, mRNA, etc. injected with genes that code for anti-inflammatory substances, etc., and, as mentioned above, osteoclasts that modify or soften bone when so desired, cells that participate in or effect mucogenesis or ciliagenesis, etc.

Additionally or alternatively to being combined with a device and/or a substance releasing modality, it may be ideal to position the device in a specific location upstream in the mucous flow path (i.e. frontal sinus or ethmoid cells). This could allow the deposition of fewer drug releasing devices, and permit the "bathing" of all the downstream tissues with the desired drug. This utilization of mucous as a carrier for the drug may be ideal, especially since the concentrations for the drug may be highest in regions where the mucous is retained; whereas non-diseased regions with good mucous flow will be less affected by the drug. This could be particularly useful in chronic sinusitis, or tumors where bringing the concentration of drug higher at those specific sites may have greater therapeutic benefit. In all such cases, local delivery will permit these drugs to have much less systemic impact. Further, it may be ideal to configure the composition of the drug or delivery system such that it maintains a loose affinity to the mucous, permitting it to distribute evenly in the flow. Also, in some applications, rather than a drug, a solute such as a salt or other mucous soluble material may be positioned at a location whereby mucous will contact the substance and a quantity of the substance will become dissolved in the mucous thereby changing some property (e.g. pH, osmolarity, etc.) of the mucous. In some cases, this technique may be used to render the mucous hyperosmolar so that the flowing mucous will draw water and/or other fluid from polyps, edematous mucosal tissue, etc., thereby providing a drying or desiccating therapeutic effect.

The above-described treatments of the Eustachian tube of a patient allow for advancing a treatment device through the guide catheter toward the Eustachian tube to place a distal tip of the treatment device adjacent the Eustachian tube opening. It may be preferred for the treatment device to have distal radiopaque member. The treatment device may include a catheter.

Alternatively or in addition, the treatment device can include a fluid introduction device for introducing a fluid into a middle ear space of the patient's ear. The fluid may be air, a contrast medium, an aspiration fluid, or a drug such as those described above. The treatment method can also include scanning the middle ear space using an ultrasound device. Alternatively, or in addition, the treatment device can include an aspiration device for aspirating a substance from the middle ear space.

Alternatively or in addition, the treatment may also include introducing a protective device proximal the Eustachian tube, and monitoring advancement of the treatment device using the protective device. The protective device may be a sensor positioned proximal the tympanic membrane to sense the position of the treatment device during the advancement. Alternatively, the protective device may comprise an endoscope to visualize the advancement.

Alternatively, or in addition, the method for treating a Eustachian tube in a patient includes placing a dual lumen pressure equalization tube through the tympanic membrane of the patient, the tube having a distal extension for location in a region of the Eustachian tube; providing a medication to the region of the Eustachian tube through a first lumen of the dual lumen tube in fluid communication with the distal extension; and providing ventilation across the tympanic membrane through a second lumen of the dual lumen tube. The medication is used to reduce edema in the Eustachian tube region.

The medication may also include surfactant configured to modify a surface tension of a mucosal layer of the Eustachian tube to effect an enhanced wetting of the mucosal surface with the medication. The medication may also include particles that are used for capturing by mucosal tissue of the Eustachian tube to effect an extended release of the medication. Exemplary surfactants are disclosed in U.S. Patent No. 6,616,913, entitled "Composition and Method for Treatment of Otitis Media".

The present disclosure also provides an apparatus for treating a Eustachian tube in a patient. The apparatus includes a dual lumen tube for insertion into a tympanic membrane of the patient's ear. The tube can include a distal extension for placement in a region of the Eustachian tube, a first lumen for providing a medication to the region of the Eustachian tube through the distal extension, and a second lumen for providing ventilation across the tympanic membrane.

The first lumen may be disposed within the second lumen. Alternatively, the second lumen is disposed within the first lumen. Additionally or alternatively, the first lumen is disposed adjacent the second lumen. The dual lumen tube may be made from or it may include a biodegradable bioresorbable material.

The Eustachian tube may be treated by delivering a drug to the Eustachian tube. An exemplary method comprises accessing a Eustachian tube region via the nasopharynx, using a guide having a lumen; introducing a guidewire through the lumen of the guide to position it submucosally between cartilage and a mucosal surface of the Eustachian tube; passing a temporary intraluminal implant having a drug delivery reservoir along the guidewire to position the implant submucosally in a posterior cushion of the Eustachian tube region between the lumen and the cartilage; and delivering a drug to the Eustachian tube region from the drug delivery reservoir.

In addition, the method may also include contemporaneously delivering a drug to adenoids and the Eustachian tube region from the drug delivery reservoir. In one embodiment, the drug delivery reservoir can comprise a coating layer disposed on the implant. In another embodiment, the guide comprises a biodegradable bioresorbable material.

The treatment of the Eustachian tube in a patient may includes obtaining access to a Eustachian tube region via the nasopharynx, introducing via the patient's nasopharynx a hollow guidewire dimensioned to reach into the Eustachian tube region, the hollow guidewire comprising a plurality of apertures disposed at or near its distal end, and delivering a drug to at least one of the Eustachian tube or a middle ear region of the patient's ear through the apertures.

The present disclosure also provides a system for accessing a Eustachian tube of a patient. The system can include a guide configured for passing into a nasal passage of the patient to position a distal tip of the catheter at or near a Eustachian tube, the guide having a distal tip with a bend having an angle between 30 and 90 degrees; and a guidewire configured to pass through the guide into the Eustachian tube.

The guide may comprise a catheter. The guide may comprises a dual lumen tube. The system may also include a diagnostic device configured for passage through the guide. The system may also include a treatment device configured for passage through the guide.

### Non-guidewire devices

Fig. 15A shows a device 1500 for treating a Eustachian tube, according to one embodiment. The device 1500 includes an elongate rigid shaft 1502. The rigid shaft may be constructed from a semi-flexible metal or plastic. "Rigid" as used with regards to device 1500 means that the shaft 1502 will not deform when inserting the shaft 1502 into a nasal cavity. The rigid shaft 1502 may be formed from a malleable material, and custom bent for use in the field. A therapeutic device, which in this example is a elongate flexible insert 1504, is coupled to the distal portion of the rigid shaft 1502. A stop (not shown) may be placed at the insert 1504 / shaft 1502 junction to prevent the shaft from entering a Eustachian tube. The insert 1504 preferentially includes a lateral stiffness such that when inserted into a Eustachian tube, the insert 1504 will conform to the pathway of the Eustachian tube and not cause significant deformation of the Eustachian tube. The insert 1504 may also include a preformed shape (not shown), for example which is preformed to the anatomy of a Eustachian tube. The insert 1504 preferentially includes a column stiffness strong enough to insert into a Eustachian tube without collapsing on itself or buckling. This example of an insert 1504 includes a core wire 1506 and an expandable balloon 1508. The core wire 1506 may be constructed from metal, such as stainless steel, or a super-elastic alloy such as nickel-titanium. Core wire 1508 diameters in the range of .05-.25mm may be suitable. The balloon 1508 may be of compliant, semi-compliant, or non-compliant construction. The balloon 1508 may include a preformed shape which matches the profile of a Eustachian tube. The balloon 1508 may include micropores for delivery, upon partial or full expansion, of any of the therapeutic substances disclosed herein. The balloon 1508 may include a coating for delivery of any of the therapeutic substances disclosed herein. The device 1500 may include an atraumatic tip 1510 in the shape of a ball, which may be integral to the core wire 1506. The device 1500 may include a fitting 1511 at the proximal portion of the shaft 1502 for supplying fluid, energy and electrical signals to the insert 1504. The device 1500 may accordingly include a lumen for passage of fluids. The device 1500 does not require a guidewire for insertion into a Eustachian tube, however a guidewire may be optionally used.

The device 1500 may be manually inserted by grasping the shaft 1502 and guiding the insert into a nasal passage and nasopharynx, and into the Eustachian tube, by way of a scope, fluoroscopic, or transillumination. Accordingly, portions of the device 1500 may include radiopaque coatings or materials. The insert 1504 may include fiber optics for transmitting light for transillumination. Examples of transilluminating devices are shown in co-assigned U.S. Patent Applications No. 10/829,917 and No. 11/522,497. The insert 1504 may also include a CCD or CMOS camera, and associated wiring, for endoscopic viewing without a separate scope. The device 1500 may also be linked to a 3-D tracking system.

The insert 1504 shown is merely an example, and may include other constructions, such as a bare wire. The bare wire may deliver energy, for example resistive heat, ultrasonic, or electrosurgical energy (e.g. RF). Energy may also be delivered by the balloon 1504, for example by a hot fluid or gas.

The insert 1504 may also deliver a stent for supporting or expanding the Eustachian tube. The stent may include a polymer material, which may elute any of the therapeutic substances disclosed herein.

The insert 1504 may also be detachable from the shaft 1504 for delivery into the Eustachian tube. In one example, the insert 1504 may be constructed from a biodegradable polymer, such as polylactic acid, which may also include any of the therapeutic substances disclosed herein. The insert 1504 may then degrade over time and deliver a therapeutic substance as required. The biodegradable insert 1504 may also include a lumen for drainage of fluid in the Eustachian tube.

Fig. 15B shows an alternative device 1512 for treating a Eustachian tube, according to one embodiment. The device 1512 is largely constructed as shown in Fig. 15A, however this embodiment includes a rigid shaft 1514 which includes a preferential bend 1516. The bend 1516 may range from 30 to 90 degrees. The bend 1516 allows for easier access to the Eustachian tube in certain anatomies.

Fig. 15C shows the device 1500 or 1512 in use, according to one embodiment. The device 1500 is shown with the insert 1504 placed within a Eustachian tube ET. The insert 1504 preferentially deforms to match the profile of the Eustachian tube ET, and thus may deliver a therapy without deforming or damaging the Eustachian tube ET. Alternatively, the insert 1504 is preformed to match the profile of the Eustachian tube and deforms slightly while being positioned. The insert 1504 also includes a column stiffness which is significant enough to prevent buckling of the insert during insertion into the Eustachian tube ET, and thus prevent damage to the device or Eustachian tube ET.

Fig. 15D shows the device 1500 or 1512 in use, according to one embodiment. In this embodiment the device 1500 includes a stent 1518 which may be expanded within the Eustachian tube ET. The stent may include a shape-memory alloy construction or a deformable construction which is expanded by the balloon 1508.

Fig. 15D shows the device 1500 or 1512 in use, according to one embodiment. In this embodiment the device 1500 includes a detachable insert 1520. The detachable insert may be detached at junction 1522. In this example, the insert 1520 includes a lumen. The insert 1520 may be biodegradable and deliver a therapeutic substance over time.

Figs. 16A and 16B show a method for providing therapy to a Eustachian tube of a patient, according to one embodiment. A guide catheter 1600 is routed through a nasal passage of a patient and placed adjacent to the opening of a Eustachian tube ET. A distal portion 1602 of the guide catheter 1600 includes a bend having an angle between 30 and 90 degrees. In one embodiment, the distal portion 1602 may be more flexible than the proximal portion of the guide catheter 1600. In one embodiment, the distal portion 1602 of the guide catheter may be malleable. Accordingly, a user may bend the distal portion 1602 to place the guide catheter 1600 in a desired position with relation to the Eustachian tube ET. A guidewire 1604 can then be advanced through the guide catheter 1600 and into the Eustachian tube ET.

In Fig. 16B a dilation catheter 1606 is advanced over the guidewire 1600 to position a dilator 1608 of the dilation catheter 1606 within the Eustachian tube ET. The guide catheter 1600 may be optionally removed from the patient before advancing the dilation catheter 1606 over the guidewire 1604. The dilation catheter 1606 generally includes an elongate shaft and the dilator 1608. The dilator 1608 may be a polymer balloon (compliant, semi-compliant or non-compliant). In some embodiments, the balloon may be porous, to deliver a therapeutic or diagnostic agent when pressurized. Alternatively, the dilator 1608 may be a mechanically expandable basket constructed from a plurality of metal or polymer tines. The dilation catheter 1606 generally includes proximally located connections/provisions (not shown) for inflating/activating the dilator 1608. A therapeutic or diagnostic agent may be applied to the interior of the Eustachian tube ET before or after the insertion of the dilation catheter 1606, for example, via a spray catheter or a spray lumen of the guide catheter 1600.

The dilator 1608 may be expanded to dilate the Eustachian tube ET after it is placed in a desirable location therein. The opening area of the Eustachian tube ET includes a pharyngeal ostium, and the dilation catheter 1606 may be advanced to position the dilator 1608 in the pharyngeal ostium. An endoscope may be used to assist in positioning the dilation catheter 1606. The endoscope may be advanced through the nasal passage to view the dilation catheter 1606. A marker on a shaft of the dilation catheter 1606 can be viewed from the endoscope to approximate a location of the dilator 1608 relative to the opening of the Eustachian tube ET based on a distance of the marker from a proximal end of the dilator 1608. Accordingly, the dilation catheter 1606 can be moved to place the marker in a desirable location before expansion of the dilator 1608 in the Eustachian tube ET.

The dilator 1608 may be held in location while in an expanded state for an extended period of time (e.g. several seconds or minutes). The dilator 1608 may also deliver a substance to the Eustachian tube ET, such as one or more of the therapeutic or diagnostic agents described herein. The dilator 1608 may also carry an expandable stent for delivery into the Eustachian tube upon expansion of the dilator 1608. The dilation catheter 1606 and the guidewire 1600 may be removed from the patient after the dilator is 1608 has been unexpanded.

Fig. 17A shows an illuminated guidewire 1700 in use, according to one embodiment. The illuminated guidewire 1700 is used in the same manner as the guidewire 1604 described above. However, the illuminated guidewire 1700 provides illumination at a distal tip 1702, which is visible to a user on the external face of the patient (commonly referred to as "transillumination"). The user may place the distal tip 1702 at a desired location based on the position of a light point 1704 passing through the patient's tissue. The light point 1704 may be used as a point of reference for the placement of other devices based on the relative distance from the light point 1704. The light point 1704 may also provide a secondary or primary light source for an endoscope which is viewing the pharyngeal ostium of the Eustachian tube ET. The illuminated guidewire 1700 may include a fiber optic channel for passing light from a light source (not shown) to the distal tip 1702. Examples of illuminated guidewires and scopes which may be used in conjunction with this disclosure are shown and described in commonly assigned U.S. Patent Application No. 11/522,497.

Figs. 18A and 18B show a dilation catheter 1800 according to an alternative embodiment of the dilation catheter 1606. The dilation catheter 1800 includes a detachable dilator 1802. The detachable dilator 1802 may be detached from the dilation catheter 1800 after the detachable dilator 1802 has been expanded within the Eustachian tube ET. The detachable dilator 1802 may include a one-way valve which allows the detachable dilator 1802 to remain dilated after detachment from the dilation catheter 1800. A breakable joint may join the detachable dilator 1802 and the dilation catheter 1800. The detachable dilator 1802 may include at least one lumen to allow the passage of a pressurizing fluid. In use, the dilation catheter 1800 is used as described herein with respect to the dilation catheter 1606. After the detachable dilator 1802 is inflated, it may be detached at the breakable joint via a twisting or pulling force applied at the proximal portion of the dilation catheter 1800. The one-way valve prevents the detachable dilator 1802 from deflating after detachment from the dilation catheter 1800. The detachable dilator 1802 may include a therapeutic or diagnostic agent to treat the Eustachian tube ET. Pressure within the Eustachian tube ET may be balanced via the lumen while the detachable dilator 1802 remains dilated therein. The detachable dilator 1802 may be removed by pulling it out of the Eustachian tube ET, and optionally puncturing the detachable dilator 1802.

Fig. 18C shows a dilation catheter 1804 according to an alternative embodiment of the dilation catheter 1606. The dilation catheter 1804 includes a plurality of extendable needles 1806, which may extend through passages in the dilator 1808. Each needle 1806 can be fluidly connected to a therapeutic or diagnostic agent source, such as a syringe. Different needles 1806 can be connected to different kinds of therapeutic or diagnostic agents. In use, the dilation catheter 1800 is used as described herein with respect to the dilation catheter 1606. After the dilator 1808 is inflated, the needles 1806 may be advanced through the dilator 1808 and into tissue of the Eustachian tube ET. The needles 1806 may then inject one or more kinds of therapeutic or diagnostic agents into the tissue of the Eustachian tube ET as shown by the substance plumes P. After the substance has been injected into the tissue of the Eustachian tube ET, the needles may be withdrawn back into the dilation catheter 1804 and the dilation catheter 1804 may be removed from the Eustachian tube ET.

Fig. 18D shows a dilation catheter 1810 according to an alternative embodiment of the dilation catheter 1606. The dilation catheter 1810 includes at least one pair of opposed lateral wings 1812 which help maintain the position of the dilator 1814 in the Eustachian tube ET. More than one pair of opposed lateral wings 1812 may be used. The lateral wings 1812 do not have to be positioned directly opposite each other, and configurations of odd-numbered lateral wings 1812 may be used. The lateral wings 1812 can be constructed from elongate tines which are spring biased to expand when advanced out of the shaft 1816 of the dilation catheter 1810. Withdrawing the slidably housed lateral wings 1812 will cause them to collapse within the shaft 1816. The lateral wings 1812 can be manipulated at the proximal end of the dilation catheter 1810, for example, through actuation of a slider mechanism. The lateral wings 1812 can include spikes or grips to help maintain immoveable contact with the Eustachian tube ET. In use, the dilator 1814 and lateral wings 1812 are advanced out of the shaft 1816 simultaneously, or non-simultaneously (i.e. the lateral wings 1812 may be advanced before or after the dilator 1814). The lateral wings 1812 apply force to the walls of the opening of the Eustachian tube ET, which helps maintain the dilator 1814 in a desired position. The lateral wings 1812 may be withdrawn back into the shaft 1816 after the dilator 1814 has applied the desired therapy to the Eustachian tube ET.

Figs. 18E, 18F and 18G show alternative embodiments of the dilator 1608. The dilator 1818 may have a shape, as shown in Fig. 18E, which matches the conical aperture of the pharyngeal ostium of the Eustachian tube ET, to enhance dilation thereof. The dilator 1820 may also have a variable shape, such as the stepped shape shown in Fig. 18F. The dilators 1818/1820 can be attached to shafts 1822 which have lumens to balance pressure within the Eustachian tube ET. Additional pressure relief holes 1824 can be included on the shaft 1822 and/or balloons of the dilators 1818/1820 to provide pressure relief. The dilators 1818/1820 may also have a non-circular cross-section such as the "+" shape shown in Fig. 18G. This configuration provides additional pressure relief as the inflated dilators 1818/1820 do not contact and occupy the entirety of the interior of the Eustachian tube ET, as shown. Thus, pressure may be relieved through the unoccupied sections 1824 of the Eustachian tube ET.

Fig. 18H shows a dilator 1826 according to an alternative embodiment. The dilator 1826 includes cutting members 1828 circumferentially placed around the exterior of the dilator 1826. The cutting members 1828 may be wires or sharpened blades. The cutting members may be configured to deliver energy (e.g. RF). In use, the cutting members 1828 expand with the dilator to impinge on the Eustachian tube ET, which allows the dilator to open and stretch along controlled locations. Figs. 18I and 18J show before and after representations of a treated Eustachian tube ET. Cutting the Eustachian tube ET along controlled sections 1829 allows the Eustachian tube ET to maintain an expanded shape by at least partially defeating the elastic response of the Eustachian tube ET wall.

Fig. 19A shows a stent 1900 according to one embodiment. The stent 1900 is configured as a tapered coil which gradually increases in diameter from a distal portion 1902 to a proximal portion 1904. The shape of the coil may be similar in scale to the pharyngeal ostium of the Eustachian tube ET, to enhance dilation thereof. The stent 1900 may be constructed from a malleable or shape-memory alloy. Alternatively, the stent 1900 may be constructed from a biodegradable polymer. The stent 1900 may be configured to carry and deliver a substance, such as any of the therapeutic or diagnostic agents disclosed herein, for example, via a biodegradable polymeric coating containing the substance. The polymeric coating may comprise a substance matrix blended with a biodegradable polymer based on lactic or glycolic acid, or on other materials, including poly(dioxanone), poly(trimethylene carbonate) copolymers, and poly (-caprolactone) homopolymers, copolymers polyanhydride, polyorthoester, or polyphosphazene. The stent 1900 may be carried and delivered by a dilation catheter, such as any of the dilation catheters disclosed herein. In use, the stent 1900 maintains mechanical expansion of the opening of the Eustachian tube ET, as shown. Alternatively, the stent 1900 may be configured to apply a minimal force against the Eustachian tube ET wall in order to provide mechanical assistance thereto. The stent may be placed in the Eustachian tube ET permanently or removed at a later time.

Fig. 19B shows a stent 1906 according to another embodiment. The stent includes a connecting member 1908 which connects a plurality of expandable tines 1910. The tines 1910 may be constructed from a malleable or shape-memory alloy. Alternatively, the tines 1910 may be constructed from a biodegradable polymer. The tines 1910 may be configured to carry and deliver a substance, such as any of the therapeutic or diagnostic agents disclosed herein, for example, via a biodegradable polymeric coating containing the substance. The polymeric coating may comprise a substance matrix blended with a biodegradable polymer based on lactic or glycolic acid, or on other materials, including poly(dioxanone), poly(trimethylene carbonate) copolymers, and poly (-caprolactone) homopolymers, copolymers polyanhydride, polyorthoester, or polyphosphazene. The tines 1910 may be carried and delivered by a dilation catheter, such as any of the dilation catheters disclosed herein. The tines 1910 may be configured to radially self-expand upon removal from a constricting shaft, or through force from a balloon. In use, the stent 1906 maintains mechanical expansion of the Eustachian tube ET, as shown. Alternatively, the stent 1906 may be configured to apply a minimal force against the Eustachian tube ET wall in order to provide mechanical assistance thereto. The stent 1906 may be placed in the Eustachian tube ET permanently or removed at a later time.

Figs. 19C and 19D show a stent 1910 according to another embodiment. The stent includes a distal connecting member 1912 and a removable proximal connecting member 1914 which connect a plurality of expandable tines 1916. The tines 1916 may be constructed from a shape-memory alloy. Alternatively, the tines 1916 may be constructed from a biodegradable polymer. The tines 1916 may be configured to carry and deliver a substance, such as any of the therapeutic or diagnostic agents disclosed herein, for example, via a biodegradable polymeric coating containing the substance. The polymeric coating may comprise a substance matrix blended with a biodegradable polymer based on lactic or glycolic acid, or on other materials, including poly(dioxanone), poly(trimethylene carbonate) copolymers, and poly (-caprolactone) homopolymers, copolymers polyanhydride, polyorthoester, or polyphosphazene. The tines 1916 may be carried and delivered by a dilation catheter, such as any of the dilation catheters disclosed herein. The tines 1916 may be configured to radially self-expand upon removal from a constricting shaft. In use, the stent 1906 is delivered via a delivery catheter. The removable proximal connecting member 1914 can then be removed to expand the proximal portion of the stent 1910, and accordingly expand the pharyngeal ostium of the Eustachian tube ET. Once in place, the stent 1906 maintains mechanical expansion of the Eustachian tube ET. Alternatively, the tines 1916 may be configured to apply a minimal force against the Eustachian tube ET wall in order to provide mechanical assistance thereto. The stent 1910 may be placed in the Eustachian tube ET permanently or removed at a later time.

Figs. 20A, 20B and 20C show distal configurations of the guide catheter 1600 according to various embodiments. The distal tips shown can be configured to enter the Eustachian tube ET in order to enable other devices to advance therein. Fig. 20A shows a beveled tip 2002, which allows easier entry into the Eustachian tube ET via a reduced leading edge. Fig. 20B shows a tapered tip 2004, which allows easier entry into the Eustachian tube ET via a reduced leading edge. Fig. 20C shows a bulbous tip 2006, which enables the guide catheter 1600 to seal the Eustachian tube ET via an increased sealing area. The tips may be constructed from a flexible material, such as silicone or rubber, which provides good sealing ability with the Eustachian tube ET. In use, the tips can seal the Eustachian tube ET for therapies such as pressurization, suction and/or the application of a substance to the Eustachian tube ET.

Figs. 21A and 21B show an insert 2100 according to one embodiment. The insert includes a central elongate shaft 2102 with a plurality of braces 2104 circumferentially extending therefrom. Each brace 2104 is connected to an outer member 2106 which is rounded for placement against the Eustachian tube ET. The insert 2100 as shown includes three triangulated braces 2104, however, two or more braces 2104 may be used in alternative embodiments. The insert 2100 may be constructed from a flexible polymer which is extruded from a die. Alternatively, the insert 2100 may be constructed from a biodegradable polymer. The outer members 2106 may be configured to carry and deliver a substance, such as any of the therapeutic or diagnostic agents disclosed herein. The insert 2100 may be carried and delivered by a dilation catheter, such as any of the dilation catheters disclosed herein. The insert 2100 may be configured to self-expand upon removal from a constricting shaft. In use, the stent 1906 is delivered via a delivery catheter. Once in place, the insert 2100 maintains mechanical expansion of the Eustachian tube ET, as shown in Fig. 21B. The insert 2100 provides and maintains open spaces 2108 in the Eustachian tube ET to maintain pressure equalization therein. The insert 2100 may be placed in the Eustachian tube ET permanently or removed at a later time.

Fig. 22A shows a string insert 2200 according to one embodiment. The string insert 2200 may be an elongate alloy or polymer string configured to carry and deliver a substance to the Eustachian tube ET, such as any of the therapeutic or diagnostic agents described herein. The string insert 2200 may be a biodegradable polymer based on lactic or glycolic acid, or on other materials, including poly(dioxanone), poly(trimethylene carbonate) copolymers, and poly (-caprolactone) homopolymers, copolymers polyanhydride, polyorthoester, or polyphosphazene. The string insert may be flexible to conform to the passage of the Eustachian tube ET. The string insert 2200 can be made from several strings in a braided configuration. The string insert can include a proximal loop 2201 to aid in removal from the Eustachian tube ET.

Figs. 22B, 22C and 22D show delivery catheters for delivering the string insert 2200 to a Eustachian tube ET, according to various embodiments. Delivery catheter 2202 is configured as a shaft and includes a snare 2204 for externally holding the string insert 2200. The delivery catheter 2202 can be configured to be slid over the guidewire 1604. In use, the snare 2204 may be actuated to release tension on the string insert 2200 and allow the removal thereof from the delivery catheter 2202.

Delivery catheter 2204 is configured as a shaft which externally holds the string insert 2200, with a distal portion of the string insert 2200 being internally located. A slidable cutting member 2206 is moveably housed within the delivery catheter 2204. The delivery catheter 2204 can be configured to slide over the guidewire 1604. In use, the slidable cutting member 2206 moves in a distal direction to cut string insert 2200 for detachment from the delivery catheter 2204.

Delivery catheter 2208 is configured as a shaft which externally holds the string insert 2200 on an external surface of the delivery catheter 2208. The delivery catheter 2208 can be configured to slide over the guidewire 1604. A connection 2210 between the delivery catheter 2208 and the string insert 2200 can be electrically fused. In use, the connection 2210 breaks when a suitable electrical current is passed therethrough.

Figs. 22E and 22F show the string insert 2200 in use, according to one embodiment. The string insert 2200 is delivered via a delivery catheter 2212 and the guide catheter 1600. The guidewire 1604 may also be used to assist delivery. The delivery catheter 2212 may be any of the delivery catheters described above. Once the string insert 2200 is placed within the Eustachian tube ET, it can deliver a substance over a sustained period of time. The string insert 2200 may be left in the Eustachian tube ET permanently or removed at a later time.

The present invention may be embodied in other specific forms without departing from the essential characteristics thereof. These other embodiments are intended to be included within the scope of the present invention, which is set forth in the following claims.

## Claims

1. A system for dilating a Eustachian tube of a patient, the system comprising:
a guide catheter (1600, 3250) advanceable through a nasal passage of the patient and including a distal end positionable at or near an opening of the Eustachian tube of the patient, wherein a distal portion of the guide catheter includes a bend having an angle between 30 and 90 degrees, and wherein the distal portion is more flexible than a proximal portion of the guide catheter;
a guidewire (1604) advanceable through the guide catheter such that a distal end of the guidewire can enter the Eustachian tube; and
a dilation catheter (1606, 1800) advanceable over the guidewire and including a dilator (1608, 1802, 1808) positionable within and for expanding the Eustachian tube,
**characterised in that** the dilation catheter further comprises a marker on a shaft of the dilation catheter to identify a distance of the marker from a proximal end of the dilator, wherein the marker can be viewed from an endoscope to approximate a location of the dilator relative to the opening of the Eustachian tube based on the distance of the marker from the proximal end of the dilator.

2. The system of claim 1, wherein the distal portion of the guide catheter is malleable.

3. The system of claim 1, wherein the opening of the Eustachian tube comprises a pharyngeal ostium of the Eustachian tube, and wherein the dilator of the dilation catheter is positionable in the pharyngeal ostium.

4. The system of claim 1, wherein the guidewire comprises an illuminating guidewire.

5. The system of claim 4, further comprising an endoscope for viewing light emitted from the illuminating guidewire.

6. The system of claim 1, wherein the dilator (1802) is detachable from the dilation catheter.

7. The system of claim 1, further comprising a stent (1900) sized to be placed within the Eustachian tube.

8. The system of claim 1, wherein the dilation catheter includes lateral wings (1812) adjacent to the dilator for maintaining the position of the dilator.

9. The system of claim 1, wherein the dilator (1818) is shaped when inflated to match a conical aperture of a pharyngeal ostium of the Eustachian tube ET.

10. The system of claim 1, wherein the dilator (1818, 1820) is shaped to have a non-circular cross-section which does not occupy the entirety of the Eustachian tube.

11. The system of claim 1, wherein the dilator (1826) includes cutting members (1828).

12. The system of claim 9 or 10, wherein the dilation catheter has a hole (1824) on a shaft (1822) and/or the dilator (1818, 1820) of the dilation catheter to provide pressure relief.

13. The system of claim 1, further comprising structure for applying at least one substance to the Eustachian tube using the dilator.

14. The system of claim 13, wherein the dilator comprises a porous balloon (1608) or a balloon with a plurality of needles (1806) for delivering the substance.

15. The system of claim 1, wherein the dilation catheter (1810) includes means (1812) for applying a force against the Eustachian tube to maintain a position of the dilator (1814) during expanding.

16. The system of claim 1 comprising:
a delivery catheter (2202, 2204, 2208, 2212) advanceable through the guide catheter to place the delivery catheter within the Eustachian tube; and
an elongate substance delivery device advanceable into the Eustachian tube using the delivery catheter.

17. The system of claim 16, wherein the elongate substance delivery device is an elongate string (2200) configured to elute at least one substance.

18. The system of claim 17, wherein the elongate string (2200) is advanceable from an interior of the delivery catheter (2208).

19. The system of claim 17, wherein the elongate string (2200) is detachable from an exterior of the delivery catheter (2208).

20. The system of claim 16, wherein the elongate substance delivery device is a balloon (1508, 1608, 1802) configured to elute the substance overtime.

21. The system of claim 20, wherein the balloon (1802) can be decoupled from the delivery catheter by inflating the balloon.

22. The system of claim 21, wherein the balloon (1818, 1820) has a stepped shape to allow pressure equalization within the Eustachian tube.

23. The system of claim 16, wherein the elongate substance delivery device is an expandable stent (1900).

24. The system of claim 16, wherein the elongate substance delivery device is an elongate insert (2100) including an elongate central member connected to a plurality of braces (2104), each brace being connected to an elongate outer member (2106).

25. The system of claim 24, wherein the braces (2104) provide and maintain open spaces in the Eustachian tube to maintain pressure equalization therein.

26. The system of any preceding claim, wherein the guide catheter has a tapered tip (2004).

## Patentansprüche

1. System zum Dilatieren einer Eustachi-Röhre eines Patienten, wobei das System Folgendes umfasst:
einen Führungskatheter (1600, 3250), der durch einen Nasenkanal des Patienten vorschiebbar ist und ein distales Ende aufweist, das an oder in der Nähe einer Öffnung der Eustachi-Röhre des Patienten positionierbar ist, wobei ein distaler Abschnitt des Führungskatheters eine Biegung mit einem Winkel zwischen 30 und 90 Grad aufweist und wobei der distale Abschnitt flexibler als ein proximaler Abschnitt des Führungskatheters ist,
einen Führungsdraht (1604), der so durch den Führungskatheter vorschiebbar ist, dass ein distales Ende des Führungsdrahts in die Eustachi-Röhre eintreten kann, und
einen Dilatationskatheter (1606, 1800), der über den Führungsdraht vorschiebbar ist und einen Dilatator (1608, 1802, 1808) zum Expandieren der Eustachi-Röhre aufweist, der in dieser positionierbar ist,
**dadurch gekennzeichnet, dass** der Dilatationskatheter ferner eine Markierung an einem Schaft des Dilatationskatheters umfasst, um einen Abstand der Markierung von einem proximalen Ende des Dilatators zu ermitteln, wobei die Markierung von einem Endoskop aus eingesehen werden kann, um auf der Grundlage des Abstands der Markierung von dem proximalen Ende des Dilatators einen Ort des Dilatators bezüglich der Öffnung der Eustachi-Röhre zu approximieren.

2. System nach Anspruch 1, wobei der distale Abschnitt des Führungskatheters formbar ist.

3. System nach Anspruch 1, wobei die Öffnung der Eustachi-Röhre ein Ostium pharyngeum der Eustachi-Röhre umfasst und wobei der Dilatator des Dilatationskatheters in dem Ostium pharyngeum positionierbar ist.

4. System nach Anspruch 1, wobei der Führungsdraht einen Beleuchtungsführungsdraht umfasst.

5. System nach Anspruch 4, ferner umfassend ein Endoskop zum Prüfen von Licht, das von dem Beleuchtungsführungsdraht emittiert wird.

6. System nach Anspruch 1, wobei der Dilatator (1802) von dem Dilatationskatheter abtrennbar ist.

7. System nach Anspruch 1, ferner umfassend einen Stent (1900), der zum Platzieren in der Eustachi-Röhre bemessen ist.

8. System nach Anspruch 1, wobei der Dilatationskatheter seitliche Flügel (1812) aufweist, die dem Dilatator benachbart sind, um die Position des Dilatators beizubehalten.

9. System nach Anspruch 1, wobei der Dilatator (1818) so gestaltet ist, wenn er inflatiert ist, dass er mit einer konischen Öffnung eines Ostium pharyngeum der Eustachi-Röhre ET zusammenpasst.

10. System nach Anspruch 1, wobei der Dilatator (1818, 1820) so gestaltet ist, dass er einen nicht kreisförmigen Querschnitt hat, der nicht die Gesamtheit der Eustachi-Röhre einnimmt.

11. System nach Anspruch 1, wobei der Dilatator (1826) Schneidglieder (1828) aufweist.

12. System nach Anspruch 9 oder 10, wobei der Dilatationskatheter ein Loch (1824) an einem Schaft (1822) und/oder dem Dilatator (1818, 1820) des Dilatationskatheters hat, um für Druckentlastung zu sorgen.

13. System nach Anspruch 1, ferner umfassend eine Struktur zum Aufbringen mindestens einer Substanz in die Eustachi-Röhre unter Verwendung des Dilatators.

14. System nach Anspruch 13, wobei der Dilatator einen porösen Ballon (1608) oder einen Ballon mit einer Vielzahl von Nadeln (1806) zur Verabreichung der Substanz umfasst.

15. System nach Anspruch 1, wobei der Dilatationskatheter (1810) Mittel (1812) aufweist, um eine Kraft gegen die Eustachi-Röhre auszuüben, um eine Position des Dilatators (1814) während des Expandierens beizubehalten.

16. System nach Anspruch 1, umfassend:
einen Verabreichungskatheter (2202, 2204, 2208, 2212), der durch den Führungskatheter vorschiebbar ist, um den Verabreichungskatheter in der Eustachi-Röhre zu platzieren, und
eine längliche Substanzverabreichungsvorrichtung, die unter Verwendung des Verabreichungskatheters in die Eustachi-Röhre vorschiebbar ist.

17. System nach Anspruch 16, wobei die längliche Substanzverabreichungsvorrichtung ein länglicher Faden (2200) ist, der zum Eluieren mindestens einer Substanz ausgestaltet ist.

18. System nach Anspruch 17, wobei der längliche Faden (2200) von einem Inneren des Verabreichungskatheters (2208) vorschiebbar ist.

19. System nach Anspruch 17, wobei der längliche Faden (2200) von einer Außenseite des Verabreichungskatheters (2208) abtrennbar ist.

20. System nach Anspruch 16, wobei die längliche Substanzverabreichungsvorrichtung ein Ballon (1508, 1608, 1802) ist, der zum Eluieren der Substanz im Laufe der Zeit ausgestaltet ist.

21. System nach Anspruch 20, wobei der Ballon (1802) durch Inflatieren des Ballons von dem Verabreichungskatheter entkoppelt werden kann.

22. System nach Anspruch 21, wobei der Ballon (1818, 1820) eine gestufte Gestalt hat, um einen Druckausgleich in der Eustachi-Röhre zu gestatten.

23. System nach Anspruch 16, wobei die längliche Substanzverabreichungsvorrichtung ein expandierbarer Stent (1900) ist.

24. System nach Anspruch 16, wobei die längliche Substanzverabreichungsvorrichtung ein länglicher Einsatz (2100) ist, der ein längliches mittleres Glied aufweist, das mit einer Vielzahl von Streben (2104) verbunden ist, wobei jede Strebe mit einem länglichen äußeren Glied (2106) verbunden ist.

25. System nach Anspruch 24, wobei die Streben (2104) offene Räume in der Eustachi-Röhre bereitstellen und aufrechterhalten, um darin den Druckausgleich aufrechtzuerhalten.

26. System nach einem der vorhergehenden Ansprüche, wobei der Führungskatheter eine sich verjüngende Spitze (2004) hat.

## Revendications

1. Système permettant de dilater une trompe d'Eustache d'un patient, le système comprenant :
un cathéter de guidage (1600, 3250) pouvant être avancé à travers un passage nasal du patient et comprenant une extrémité distale pouvant être positionnée au niveau ou près d'une ouverture de la trompe d'Eustache du patient, dans lequel une partie distale du cathéter de guidage comprend un coude formant un angle entre 30 et 90 degrés, et dans lequel la partie distale est plus souple qu'une partie proximale du cathéter de guidage ;
un fil-guide (1604) pouvant être avancé à travers le cathéter de guidage de sorte qu'une extrémité distale du fil-guide puisse entrer dans la trompe d'Eustache ; et
un cathéter de dilatation (1606, 1800) pouvant être avancé sur le fil-guide et comprenant un dilatateur (1608, 1802, 1808) pouvant être positionné à l'intérieur de la trompe d'Eustache et permettant de déployer celle-ci,
**caractérisé en ce que** le cathéter de dilatation comprend en outre un repère sur un arbre du cathéter de dilatation afin d'identifier une distance du repère à partir d'une extrémité proximale du dilatateur, dans lequel le repère peut être visualisé depuis un endoscope pour s'approcher d'un emplacement du dilatateur par rapport à l'ouverture de la trompe d'Eustache sur la base de la distance du repère depuis une extrémité proximale du dilatateur.

2. Système selon la revendication 1, dans lequel la partie distale du cathéter de guidage est malléable.

3. Système selon la revendication 1, dans lequel l'ouverture de la trompe d'Eustache comprend un ostium pharyngien de la trompe d'Eustache, et dans lequel le dilatateur du cathéter de dilatation peut être positionné dans l'ostium pharyngien.

4. Système selon la revendication 1, dans lequel le fil-guide comprend un fil-guide éclairant.

5. Système selon la revendication 4, comprenant en outre un endoscope pour voir la lumière émise par le fil-guide éclairant.

6. Système selon la revendication 1, dans lequel le dilatateur (1802) peut se détacher du cathéter de dilatation.

7. Système selon la revendication 1, comprenant en outre une endoprothèse (1900) dimensionnée pour être placée à l'intérieur de la trompe d'Eustache.

8. Système selon la revendication 1, dans lequel le cathéter de dilatation comprend des ailes latérales (1812) adjacentes au dilatateur pour maintenir la position du dilatateur.

9. Système selon la revendication 1, dans lequel le dilatateur (1818), lorsqu'il est gonflé, a une forme qui correspond à une ouverture conique d'un ostium pharyngien de la trompe d'Eustache ET.

10. Système selon la revendication 1, dans lequel le dilatateur (1818, 1820) est formé pour avoir une coupe transversale non circulaire qui n'occupe pas la totalité de la trompe d'Eustache.

11. Système selon la revendication 1, dans lequel le dilatateur (1826) comprend des éléments coupants (1828) .

12. Système selon la revendication 9 ou 10, dans lequel le cathéter de dilatation possède un trou (1824) sur un arbre (1822) et/ou le dilatateur (1818, 1820) du cathéter de dilatation pour fournir un relâchement de pression.

13. Système selon la revendication 1, comprenant en outre une structure pour appliquer au moins une substance à la trompe d'Eustache à l'aide du dilatateur.

14. Système selon la revendication 13, dans lequel le dilatateur comprend un ballonnet poreux (1608) ou un ballonnet comportant une pluralité d'aiguilles (1806) pour administrer la substance.

15. Système selon la revendication 1, dans lequel le cathéter de dilatation (1810) comprend des moyens (1812) pour appliquer une force contre la trompe d'Eustache afin de conserver une position du dilatateur (1814) pendant l'expansion.

16. Système selon la revendication 1 comprenant :
un cathéter de pose (2202, 2204, 2208, 2212) pouvant être avancé à travers le cathéter de guidage pour placer le cathéter de pose à l'intérieur de la trompe d'Eustache ; et
un dispositif allongé d'administration de substance pouvant être avancé dans la trompe d'Eustache à l'aide du cathéter de pose.

17. Système selon la revendication 16, dans lequel le dispositif allongé d'administration de substance est une ficelle allongée (2200) conçue pour éluer au moins substance.

18. Système selon la revendication 17, dans lequel la ficelle allongée (2200) peut être avancée depuis une partie intérieure du cathéter de pose (2208).

19. Système selon la revendication 17, dans lequel la ficelle allongée (2200) peut se détacher d'une partie extérieure du cathéter de pose (2208).

20. Système selon la revendication 16, dans lequel le dispositif allongé d'administration de substance est un ballonnet (1508, 1608, 1802) conçu pour éluer la substance au fil du temps.

21. Système selon la revendication 20, dans lequel le ballonnet (1802) peut être désolidarisé du cathéter de pose en gonflant le ballonnet.

22. Système selon la revendication 21, dans lequel le ballonnet (1818, 1820) a une forme étagée pour permettre une égalisation de pression dans la trompe d'Eustache.

23. Système selon la revendication 16, dans lequel le dispositif allongé d'administration de substance est une endoprothèse déployable (1900).

24. Système selon la revendication 16, dans lequel le dispositif allongé d'administration de substance est un insert allongé (2100) comprenant un élément central allongé relié à une pluralité d'appuis (2104), chaque appui étant relié à un élément externe allongé (2106).

25. Système selon la revendication 24, dans lequel les appuis (2104) forment et maintiennent des espaces ouverts dans la trompe d'Eustache afin de conserver une égalisation de pression en son sein.

26. Système selon l'une quelconque des revendications précédentes, dans lequel le cathéter de guidage possède un bout conique (2004).
